# EUROPEAN PATENT APPLICATION

(11) **EP 4 063 371 A1**
(43) Date of publication of application: **28.09.2022**
(21) Application number: 20889371.9
(22) Date of filing: 20.11.2020
(51) Int. Cl.: C07D 491/20, A61K 31/519, A61K 31/496, A61P 35/00, A61P 35/02

(54) **PYRIMIDOPYRROLE SPIRO COMPOUNDS AND DERIVATIVES THEREOF AS DNA-PK INHIBITORS**

(30) Priority: 22.11.2019 CN 201911154894; 23.03.2020 CN 202010209359; 12.11.2020 CN 202011258837
(71) Applicant: Medshine Discovery Inc., Nanjing, Jiangsu 210032 (CN)
(72) Inventor: CHEN, Kevin X, Shanghai 200131 (CN); XIA, Shanghua, Shanghai 200131 (CN); CHEN, Zhaoguo, Shanghai 200131 (CN); GUO, Zuhao, Shanghai 200131 (CN); YU, Yanxin, Shanghai 200131 (CN); ZHOU, Kai, Shanghai 200131 (CN); HU, Boyu, Shanghai 200131 (CN); ZHANG, Li, Shanghai 200131 (CN); JIANG, Fen, Shanghai 200131 (CN); WANG, Jingjing, Shanghai 200131 (CN); HU, Guoping, Shanghai 200131 (CN); LI, Jian, Shanghai 200131 (CN); CHEN, Shuhui, Shanghai 200131 (CN)
(74) Representative: Dehns
(86) International application number: PCT/CN2020/130335
(87) International publication number: WO 2021/098813

(57) **Abstract**

Provided are a class of DNA-PK inhibitor, and specifically, a compound represented by formula (III) or a pharmaceutically acceptable salt thereof, and use thereof in the preparation of DNA-PK inhibitor-related drugs.

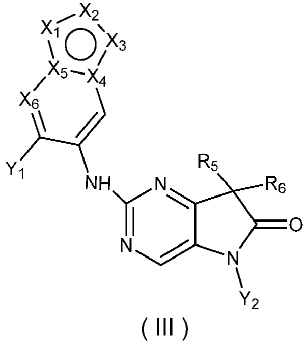

## Description

The present invention claims the following priorities:
CN201911154894.9, filed on November 22, 2019;
CN202010209359.5, filed on March 23, 2020;
CN202011258837.8, filed on November 12, 2020.

### Technical field

The present disclosure relates to a DNA-PK inhibitor, in particular to a compound represented by formula (III) or a pharmaceutically acceptable salt thereof, and a use thereof in the manufacture of a medicament related ro a DNA-PK inhibitor.

### Background

DNA breaks, especially double-strand breaks (DSBs), are extremely serious damages that can cause loss of genetic material, genetic recombination, and lead to cancer or cell death. Eukaryotic cells have evolved a variety of mechanisms to deal with the serious threat of DNA double-strand breaks, which are the DNA damage response mechanism (DDR), which mainly include DNA damage detection, signal transduction, and damage repair. DNA double-strand break repair mainly includes homologous recombination (HR) repair and non-homologous end joining (NHEJ) repair. In higher eukaryotes, NHEJ repair, preferentially used during early G1/S phase, is the main mechanism. DDR initial damage factors such as MRN detect and identify the damage site, recruit members of the phosphatidylinositol kinase family (ATM, ATR, DNA-PK), phosphorylate H2AX to promote the formation of γH2AX, guide downstream signal transduction and recruit related proteins to complete the repair of damaged DNA.

DNA-PK catalytic subunit (DNA-PKcs), belonging to the phosphoinositide-3-kinase-related protein (PI3K-related kinase, PIKK) family, is mainly for the repair of non-homologous end joining (NHEJ) of DNA double strand breaks, and is an important member of DNA damage repair. During the repair of DNA double-strand damage, the Ku70/Ku80 heterodimer specifically connects to the double-strand damage site through a pre-formed channel to identify double-strand breaks and bind to the ends of the breaks respectively. Then, the ATP-dependent manner is used to slide a distance along the DNA chain to both ends to form KU-DNA complexes and recruit DNA-PKcs to bind to the double-strand break sites. Subsequently, Ku dimer moves inward to activate DNA-PKcs and make them self-phosphorylated. Finally, phosphorylated DNA-PKcs guides damage signal transduction and recruits DNA end processing-related proteins such as PNKP, XRCC4, XLF, Pol X, and DNA ligase IV to participate in double-strand break repair.

At present, the main mechanisms of DNA damaging chemotherapeutic drugs (such as bleomycin, topoisomerase II inhibitors such as etoposide and doxorubicin) and radiotherapy commonly used in tumor therapy are to cause fatal double-strand breaks of DNA molecules, and then induce the death of tumor cells. Studies have shown that high expression of DNA-PK is found in tumor tissues treated with chemoradiotherapy, and the increase of DNA-PKcs activity to a certain extent enhances the repair of damaged DNA, prevents tumor cell death, and leads to the tolerance of chemoradiotherapy. In addition, the surviving cells in the tumor tissue after chemoradiotherapy are often cells with high DNA-PKcs activity that are not sensitive to the treatment, which are also the reason for the poor curative effect and poor prognosis. Combined with chemoradiotherapy drugs, DNA-PK inhibitors can inhibit the activity of DNA-PKcs, thereby greatly reducing tumor DNA repair, inducing cells to enter the apoptosis process, and achieving better therapeutic effects.

ATM plays an important role in homologous recombination (HR) repair, and when tumor cells are deficient in ATM, DNA break repair becomes more dependent on DNA-PKcs-dominated NHEJ repair for their survival. Therefore, DNA-PK inhibitors can also act as single drugs in tumors with defects in other DNA repair pathways.

The DNA-PK small molecule inhibitor of the present disclosure can not only play a therapeutic effect as a single drug in tumors with defects in other DNA repair pathways. It can also be combined with chemoradiotherapy drugs to enhance the sensitivity of tumor tissues to chemoradiotherapy, overcome the drug resistance problem, and enhance the inhibitory effect on various solid tumors and hematological tumors. Such compounds have good activity and show excellent effects and functions, with broad prospects.

### Content of the present invention

The present disclosure provides a compound represented by formula (III) or a pharmaceutically acceptable salt thereof, 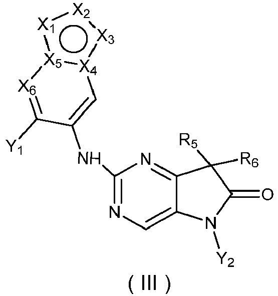 wherein,
R₅ and R₆ combining with the carbon atoms to which they are attached form 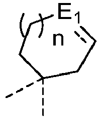 or 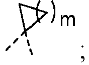
when 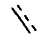 is a single bond, E₁ is selected from -O-, -S-, -C(=O)-, -S(O)₂-, -C(R₁)(R₂)-, -N(R₃)- and 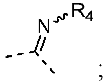
when 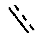 is a double bond, E₁ is selected from -C(Ri)-;
R₁ and R₂ are each independently selected from H, OH, F, Cl, Br, I, C₁₋₃ alkoxy and C₁₋₃ alkyl, and the C₁₋₃ alkoxy and C₁₋₃ alkyl are optionally substituted by 1, 2 or 3 Ra;
or, R₁ and R₂ combining with the carbon atoms to which they are attached form a cyclopropyl, cyclobutyl and oxetanyl;
R₃ is selected from C₁₋₃ alkyl-C(=O)- and C₁₋₃ alkyl, and the C₁₋₃ alkyl-C(=O)- and C₁₋₃ alkyl are optionally substituted by 1, 2 or 3 Rb;
R₄ is selected from C₁₋₃ alkoxy;
n is selected from 0, 1 and 2, provided that when E₁ is selected from -C(R₁)(R₂)-, and both R₁ and R₂ are selected from H, n is not 0;
m is selected from 1, 2 and 3;
X₁, X₂, X₃, X₄ and X₅ are each independently selected from N, C and CH, provided that at most three of X₁, X₂, X₃, X₄ and X₅ are N, and the ring formed with X₁, X₂, X₃, X₄ and X₅ is an aromatic ring;
X₆ is selected from CH and N;
Y₁ is selected from F, Cl, Br, I, cyclopropyl and C₁₋₃ alkyl, and the C₁₋₃ alkyl is optionally substituted by OH or 1, 2 or 3 Ra;
Y₂ is selected from cyclopropyl and C₁₋₃ alkyl, and the C₁₋₃ alkyl is optionally substituted by 1, 2, 3, 4 or 5 F;
Ra and R_{b} are each independently selected from H, F, Cl, Br, I.

In some embodiments of the present disclosure, the compound represented by formula (III) or the pharmaceutically acceptable salt thereof is selected from a compound represented by formula (III-1) or a pharmaceutically acceptable salt thereof, 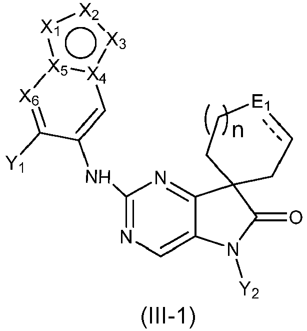 wherein, X₁, X₂, X₃, X₄, X₅, X₆, Y₁, Y₂, E₁ and n are as defined herein.

In some embodiments of the present disclosure, the compound represented by formula (III) or the pharmaceutically acceptable salt thereof is selected from a compound represented by formula (III-2) or a pharmaceutically acceptable salt thereof, 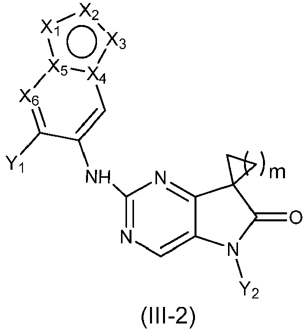 wherein, X₁, X₂, X₃, X₄, X₅, X₆, Y₁, Y₂ and m are as defined herein.

In some embodiments of the present disclosure, the X₁, X₃ and X₄ are selected from N, X₂ is selected from CH, X₅ is selected from C, and X₆ is selected from CH and N; in some embodiments of the present disclosure, the X₁, X₂ and X₄ are selected from N, X₃ is selected from CH, X₅ is selected from C, X₆ is selected from CH; in some embodiments of the present disclosure, the X₁, X₃ and X₅ are selected from N, X₂ is selected from CH, X₄ is selected from C, X₆ is selected from CH; in some embodiments of the present disclosure, the X₁ and X₄ are selected from N, X₂ and X₃ are selected from CH, X₅ is selected from C, X₆ is selected from CH and N; the other variables are as defined herein.

In some embodiments of the present disclosure, the Y₁ is selected from F, Cl, cyclopropyl, CH₃, CH₂OH, CFH₂, CF₂H and CF₃; in some embodiments of the present disclosure, the Y₂ is selected from cyclopropyl, CH₃, CFH₂, CF₂H and CF₃; the other variables are as defined herein.

In some embodiments of the present disclosure, the compound represented by formula (III) or the pharmaceutically acceptable salt thereof is selected from a compound represented by formula (I), a compound represented by formula (II) or a pharmaceutically acceptable salt thereof, 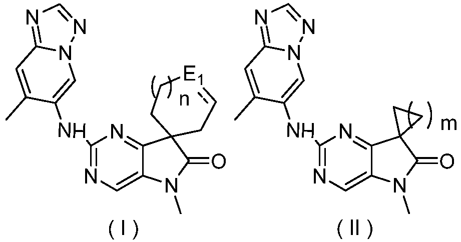 wherein, E₁, m and n are as defined herein.

In some embodiments of the present disclosure, 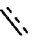 is a single bond, E₁ is selected from -O-, -S-, -C(=O)-, -S(O)₂-, -C(R₁)(R₂)-, -N(R₃)- and 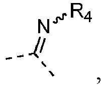 and Ri, R₂, R₃ and R₄ are as defined herein; in some embodiments of the present disclosure, E₁ is selected from -O-, - C(R₁)(R₂)-, -N(R₃)- and 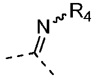 , and Ri, R₂, R₃ and R₄ are as defined herein; the other variables are as defined herein.

In some embodiments of the present disclosure, 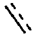 is a single bond, E₁ is selected from -O-, -S-, -C(=O)-, -S(O)₂-, -C(R₁)(R₂)-, -N(R₃)- and 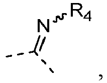 R₁ and R₂ are each independently selected from H, OH, F, Cl, C₁₋₃ alkoxy and C₁₋₃ alkyl, and the C₁₋₃ alkoxy and C₁₋₃ alkyl are optionally substituted by 1,2 or 3 H or F; R₃ is selected from C₁₋₃ alkyl-C(=O)- and C₁₋₃ alkyl, and the C₁₋₃ alkyl-C(=O)- and C₁₋₃ alkyl are optionally substituted by 1, 2 or 3 H or F; R₄ is selected from C₁₋₃ alkoxy; in some embodiments of the present disclosure, E₁ is selected from -O-, -C(R₁)(R₂), -N(R₃)- and 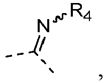 R₁ and R₂ are each independently selected from H, OH, F, Cl, C₁₋₃ alkoxy and C₁₋₃ alkyl, and the C₁₋₃ alkoxy and C₁₋₃ alkyl are optionally substituted by 1, 2 or 3 H or F, R₃ is selected from C₁₋₃ alkyl-C(=O)- and C₁₋₃ alkyl, and the C₁₋₃ alkyl-C(=O)- and C₁₋₃ alkyl are optionally substituted by 1, 2 or 3 H or F; R₄ is selected from C₁₋₃ alkoxy; the other variables are as defined herein.

In some embodiments of the present disclosure, 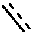 is a double bond, E₁ is selected from -C(R₁)-, R₁ is selected from H, F, Cl, Br, I, C₁₋₃ alkoxy and C₁₋₃ alkyl, and the C₁₋₃ alkoxy and C₁₋₃ alkyl are optionally substituted by 1, 2 or 3 Ra, and Ra is as defined herein; in some embodiments of the present disclosure, 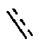 is a double bond, E₁ is selected from -C(R₁)-, R₁ is selected from H, F and C₁₋₃ alkyl, and the C₁₋₃ alkyl is optionally substituted by 1, 2 or 3 H or F; the other variables are as defined herein.

In some embodiments of the present disclosure, n is 1; in some embodiments of the present disclosure, n is 2; the other variables are as defined herein.

In some embodiments of the present disclosure, the R₁ and R₂ are each independently selected from H, OH, F, CH₃, CF₃ and CH₃O-, and the other variables are as defined herein.

In some embodiments of the present disclosure, the R₁ and R₂ combining with the carbon atoms to which they are attached form 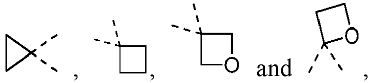 and the other variables are as defined herein.

In some embodiments of the present disclosure, the R₁ and R₂ combining with the carbon atoms to which they are attached form 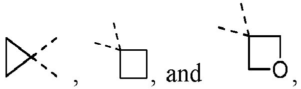 and the other variables are as defined herein.

In some embodiments of the present disclosure, the R₁ and R₂ are each independently selected from H, F, CH₃ and CH₃O-, and the other variables are as defined herein.

In some embodiments of the present disclosure, the R₁ and R₂ combining with the carbon atoms to which they are attached form 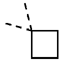 and 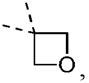 and the other variables are as defined herein.

In some embodiments of the present disclosure, the R_{b} is selected from H and F, and the other variables are as defined herein.

In some embodiments of the present disclosure, the R₃ is selected from CH₃, CH₃CH₂ and CH₃C(=O)-, and the CH₃, CH₃CH₂ and CH₃C(=O)- are optionally substituted by 1, 2 or 3 R_{b}, and the other variables are as defined herein.

In some embodiments of the present disclosure, the R₃ is selected from CH₃, CF₃CH₂ and CH₃C(=O)-, and the other variables are as defined herein.

In some embodiments of the present disclosure, the R₄ is selected from CH₃O-, and the other variables are as defined herein.

In some embodiments of the present disclosure, the structural moiety 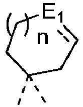 is selected from 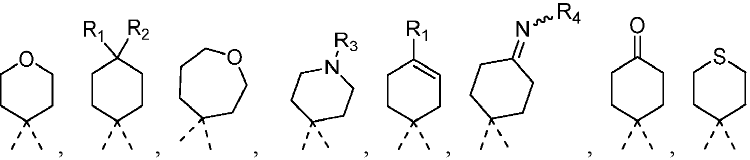 and 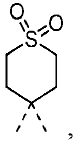 and the other variables are as defined herein.

In some embodiments of the present disclosure, the structural moiety 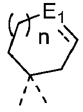 is selected from 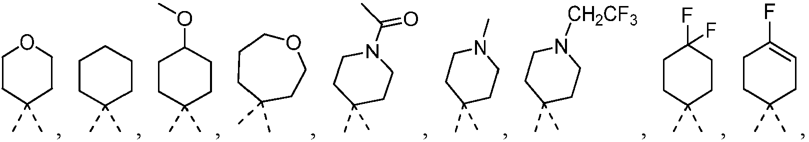 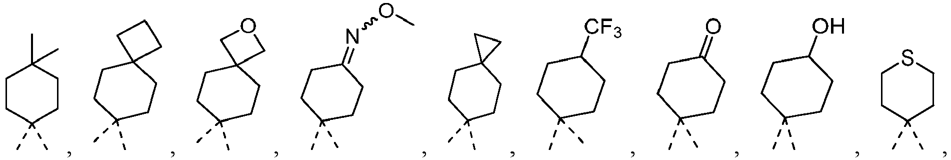 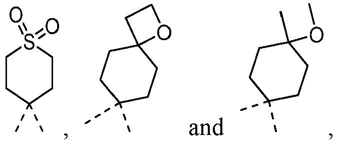 and the other variables are as defined herein.

In some embodiments of the present disclosure, the structural moiety 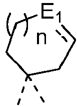 is selected from 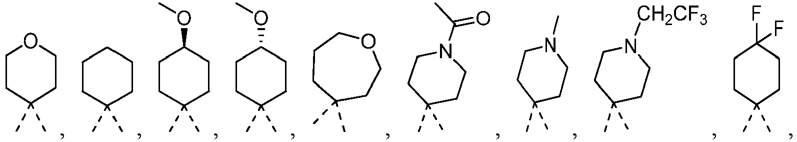 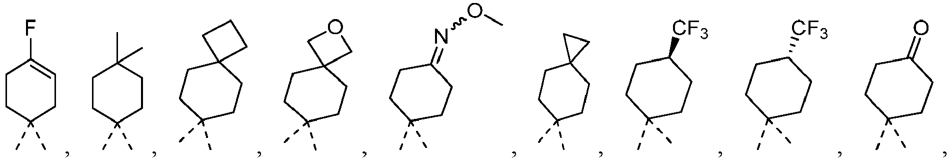 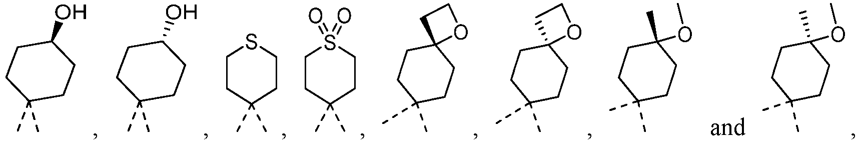 and the other variables are as defined herein.

In some embodiments of the present disclosure, the structural moiety 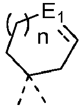 is selected from 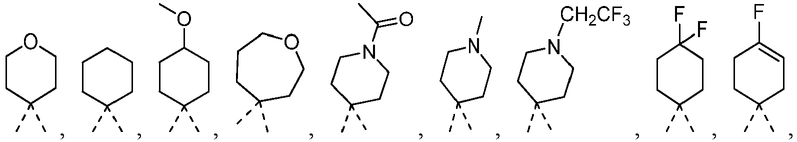 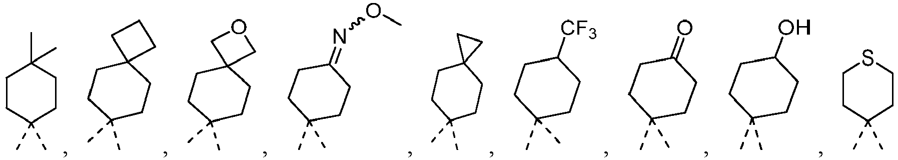 and 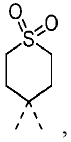 and the other variables are as defined herein.

In some embodiments of the present disclosure, the structural moiety 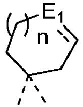 is selected from 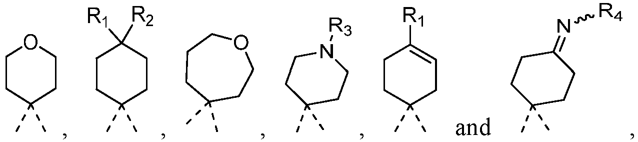 and the other variables are as defined herein.

In some embodiments of the present disclosure, the structural moiety 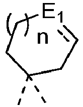 is selected from 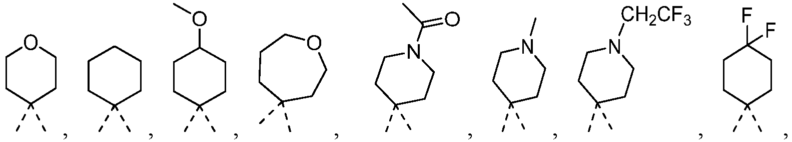 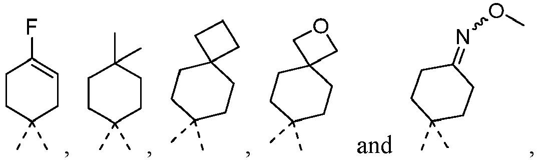 and the other variables are as defined herein.

Some embodiments of the present disclosure are formed by any combination of the above variables.

In some embodiments of the present disclosure, the compound represented by formula (III) or the pharmaceutically acceptable salt thereof is selected from 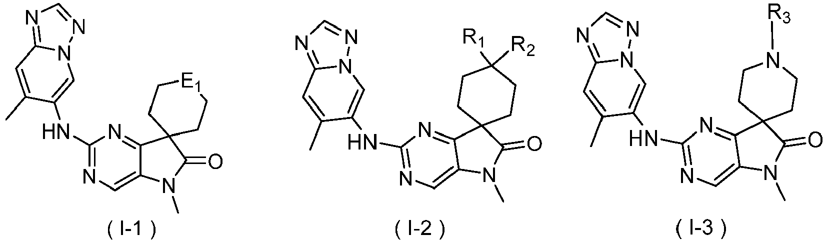 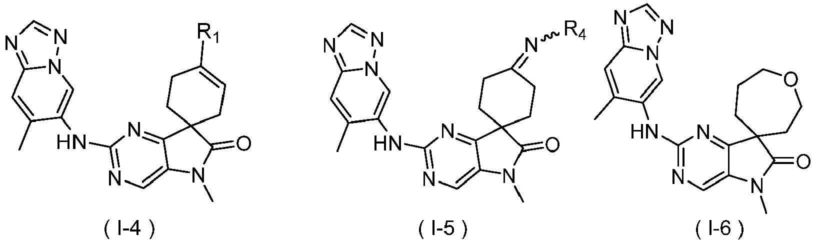 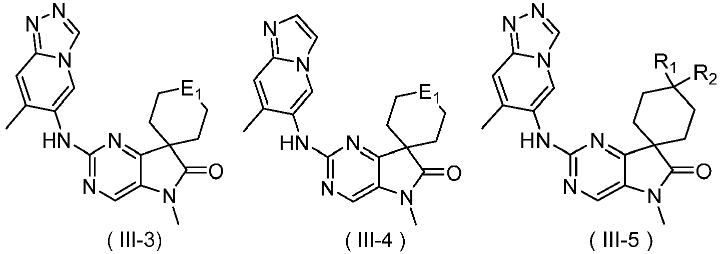 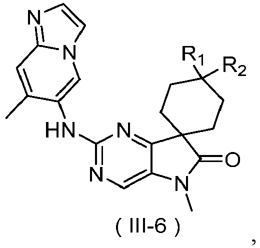 wherein E₁, Ri, R₂, R₃ and R₄ are as defined above.

The present disclosure provides a compound represented by the following formula or a pharmaceutically acceptable salt thereof.

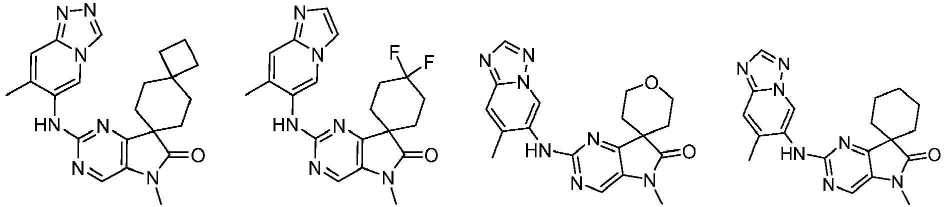

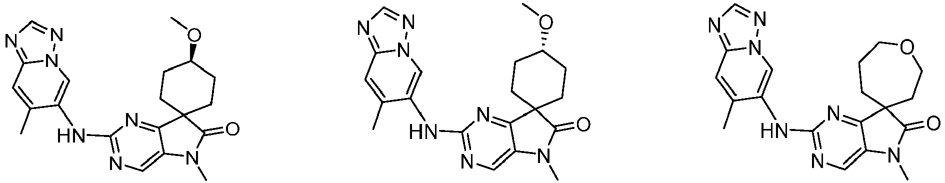

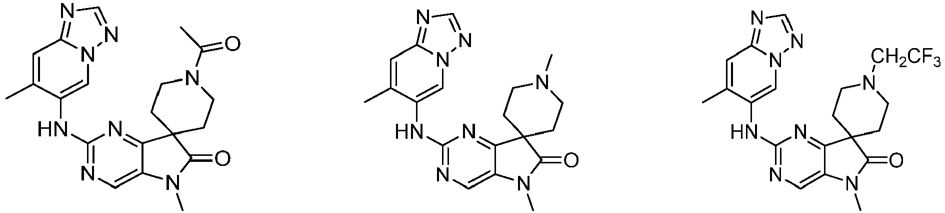

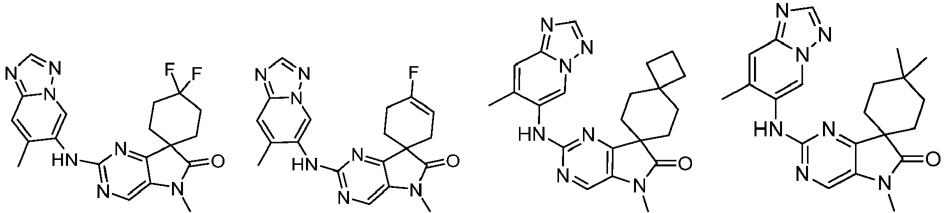

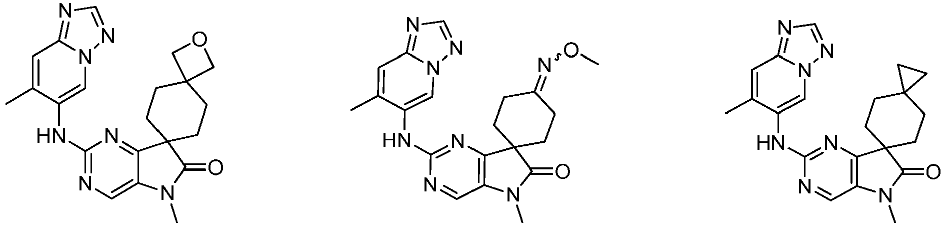

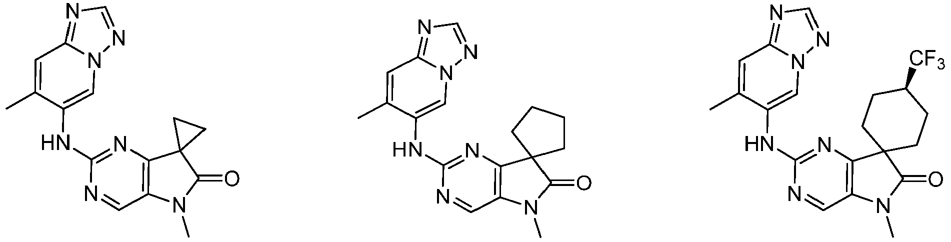

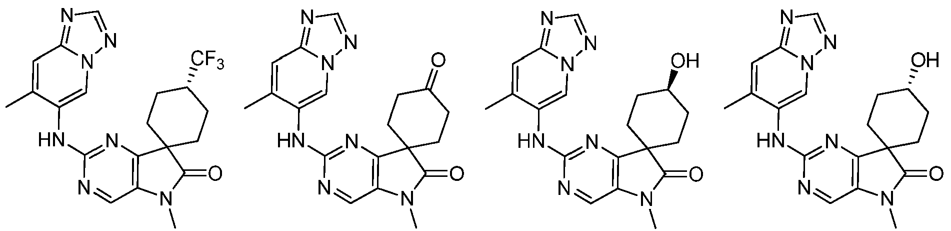

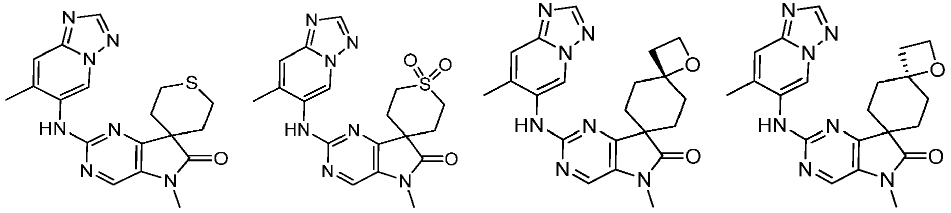

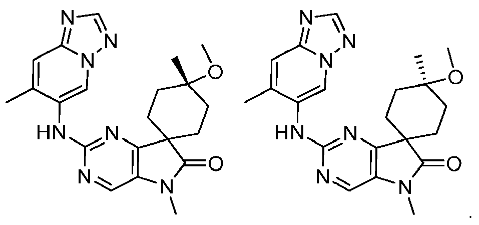

In some embodiments of the present disclosure, a use of the compound or the pharmaceutically acceptable salt thereof in the manufacture of a medicament related to a DNA-PK inhibitor.

In some embodiments of the present disclosure, the medicament related to the DNA-PK inhibitor plays a therapeutic effect as a single medicament in tumors with defects in other DNA repair pathways.

In some embodiments of the present disclosure, the medicament related to the DNA-PK inhibitor is used in combination with chemoradiotherapy medicaments to enhance the inhibitory effect on solid tumors and hematological tumors.

### Technical effects

As a class of DNA-PK inhibitors, the compound of the disclosure shows a significant DNA-PK kinase inhibitory activity. The PK results show that the compound of the present disclosure shows lower clearance rate and higher drug exposure amount, has good pharmacokinetic properties *in vivo*, and is a very good molecule capable of developing oral administration.

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)-and (+)-enantiomers, (*R*)-and (*S*)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 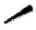 ) and a wedged dashed bond ( 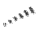 ), and the relative configuration of a stereogenic center is represented by a straight solid bond ( 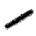 ) and a straight dashed bond ( 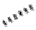 ), a wave line ( 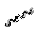 ) is used to represent a wedged solid bond ( 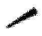 ) or a wedged dashed bond ( 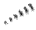 ), or the wave line ( 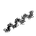 ) is used to represent a straight solid bond ( 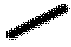 ) or a straight dashed bond ( 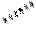 ).

Unless otherwise specified, the terms "enriched in one isomer", "enriched in isomers", "enriched in one enantiomer" or "enriched in enantiomers" refer to the content of one of the isomers or enantiomers is less than 100 %, and the content of the isomer or enantiomer is greater than or equal to 60 %, or greater than or equal to 70 %, or greater than or equal to 80 %, or greater than or equal to 90 %, or greater than or equal to 95 %, or greater than or equal to 96 %, or greater than or equal to 97 %, or greater than or equal to 98 %, or greater than or equal to 99 %, or greater than or equal to 99.5 %, or greater than or equal to 99.6 %, or greater than or equal to 99.7 %, or greater than or equal to 99.8 %, or greater than or equal to 99.9 %.

Unless otherwise specified, the term "isomer excess" or "enantiomeric excess" refers to the difference between the relative percentages of two isomers or two enantiomers. For example, if the content of one isomer or enantiomer is 90 %, and the content of the other isomer or enantiomer is 10 %, the isomer or enantiomer excess (ee value) is 80 %.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. P ositions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond.

When the number of a substituent is 0, it means that the substituent does not exist, for example, -A-(R)o means that the structure is actually A.

When a substituent is vacant, it means that the substituent does not exist, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such a substituent can be bonded to any atom on the ring, for example, a structural moiety 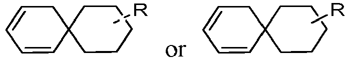 means that R can substitute on any position of cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 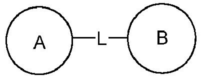 is -M-W-, then -M-W- can link ring A and ring B to form 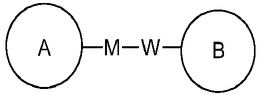 in the direction same as left-to-right reading order, and form 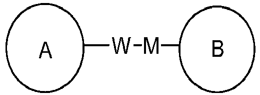 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of chemical bond linking thereto so as to meet the corresponding valence. The chemical bond between the site and other groups can be represented by a straight solid bond 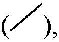 a straight dashed bond 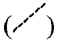 or a wavy line 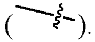 For example, the straight solid bond in -OCH₃ means that it is linked to other groups through the oxygen atom in the group; the straight dashed bonds in 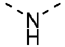 means that it is linked to other groups through the two ends of nitrogen atom in the group; the wave lines in 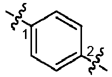 means that the phenyl group is linked to other groups through carbon atoms at position 1 and position 2; 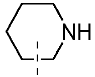 means that it can be linked to other groups through any linkable sites on the piperidinyl by one chemical bond, including at least four types of linkage, including 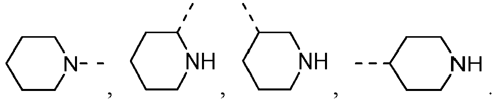 Even though the H atom is drawn on the -N-, 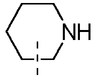 still includes the linkage of 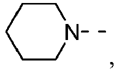 merely when one chemical bond was connected, the H of this site will be reduced by one to the corresponding monovalent piperidinyl.

Unless otherwise specified, the number of atoms in a ring is generally defined as the number of ring members, e.g., "5- to 7-membered ring" refers to a "ring" of 5-7 atoms arranged around it.

Unless otherwise specified, the term "C₁₋₃ alkyl" refers to a linear or branched saturated hydrocarbon group containing 1 to 3 carbon atoms. The C₁₋₃ alkyl group includes C₁₋₂ and C₂₋₃ alkyl groups and the like; it can be monovalent (such as methyl), divalent (such as methylene) or multivalent (such as methine). Examples of C₁₋₃ alkyl include but are not limited to methyl (Me), ethyl (Et), propyl (including *n*-propyl and isopropyl), etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃ and C₂ alkoxy, etc. Examples of C₁₋₃ alkoxy include, but are not limited to, methoxy, ethoxy, propoxy (including n-propoxy and isopropoxy), etc.

Unless otherwise specified, Cn-n+m or Cn-Cn+m includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n membered to n+m membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ scan, and after collecting the relevant data, the crystal structure can be further analyzed by direct method (Shelxs97).

The solvent used in the present disclosure is commercially available.

The present disclosure adopts the following abbreviations: eq stands for equivalent; DMSO stands for dimethyl sulfoxide, EDTA stands for ethylenediaminetetraacetic acid, DNA stands for deoxyribonucleic acid, ATP stands for adenosine triphosphate; PEG stands for polyethylene glycol; Balb/c stands for mouse strain.

The compounds of the present disclosure are named according to the conventional naming principles in the art or by ChemDraw^{®} software, and the commercially available compounds use the supplier catalog names.

### Detailed description of the preferred embodiment

The present disclosure is described in detail by the embodiments below, but it does not mean that there are any adverse restrictions on the present disclosure. The present disclosure has been described in detail herein, wherein specific embodiments thereof are also disclosed, and it will be apparent to those skilled in the art that various variations and improvements can be made to specific embodiments of the present disclosure without departing from the spirit and scope of the present disclosure.

### Embodiment 1

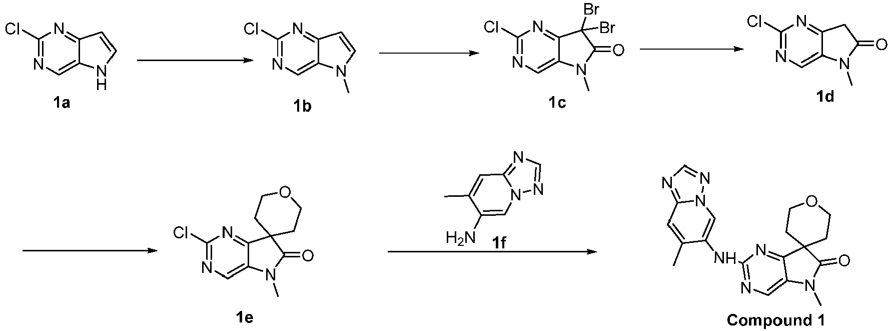

### Step 1

Sodium hydride (0.78 g, 19.5 mmol, 60 % purity, 1.3 *eq*) was added to a *N,N-*dimethylformamide (80 mL) solution of compound **1a** (2.30 g, 15 mmol, 1 *eq)* at 0 °C, and the mixture was stirred at 0 °C for 0.5 hours; then methyl iodide (2.66 g, 18.74 mmol, 1.17 mL, 1.25 *eq*) was added thereto; after the addition was completed, the reaction solution was reacted at 15 °C for 1.5 hours. After the reaction was completed, 100 mL of water was added to the reaction solution at 0 °C to quench, and the mixture was extracted with ethyl acetate (200 mL^{∗}3), washed with 80 mL of saturated brine, dried with anhydrous sodium sulfate, filtered, and the filtrate was concentrated under reduced pressure to obtain crude product **1b**. MS: *m*/*z*. 167.8 [M+H]⁺.

### Step 2

*N*-bromosuccinimide (8.01 g, 45 mmol, 3 *eq*) was added to a mixed *tert*-butanol (90 mL) and water (30 mL) solution of compound **1b** (2.51 g, 15 mmol, 1 *eq*)*,* and the reaction solution was reacted at 15 °C for 2 hours. After the reaction was completed, the reaction solution was diluted with 70 mL of water, extracted with ethyl acetate (100 mL^{∗}3), washed with 50 mL of saturated brine, dried with anhydrous sodium sulfate, filtered, and the filtrate was concentrated under reduced pressure to obtain a residue, and the obtained residue was purified by silica gel column chromatography (ethyl acetate: petroleum ether= 1: 2) to obtain compound **1c**. MS: *m*/*z.* 263.8 [M+H-Br+2]. ¹H NMR (400 MHz, CDCl₃) δ ppm 8.23 (s, 1H), 3.34 (s, 3H).

### Step 3

Zinc powder (5.23 g, 80 mmol, 20 *eq*) and acetic acid (4.80 g, 80 mmol, 4.58 mL, 20 *eq*) were sequentially added to a tetrahydrofuran (50 mL) solution of compound **1c** (1.71 g, 4 mmol, 80 % purity, 1 *eq),* and the reaction solution was reacted at 15 °C for 1 hour. After the reaction was completed, the reaction solution was diluted with 100 mL of water, extracted with ethyl acetate (100 mL^{∗}3), washed with 50 mL of saturated brine, dried with anhydrous sodium sulfate, filtered, and the filtrate was concentrated under reduced pressure and purified by silica gel column chromatography (ethyl acetate: petroleum ether= 3: 1) to obtain compound **1d**.

### Step 4

Cesium carbonate (0.782 g, 2.4 mmol, 4 *eq*) and bis(2-iodoethyl)ether (0.782 g, 2.4 mmol, 4 *eq*) were sequentially added to a *N,N*-dimethylformamide (12 mL) solution of compound **Id** (0.11 g, 0.6 mmol, 1 *eq);* after the addition was completed, the reaction solution was reacted at 60 °C for 6 hours. After the reaction was completed, the reaction solution was diluted with 30 mL of water, extracted with ethyl acetate (30 mL^{∗}3), washed with 10 mL of saturated brine, dried with anhydrous sodium sulfate, filtered, and the filtrate was concentrated under reduced pressure and purified by silica gel column chromatography (ethyl acetate: petroleum ether= 1: 1) to obtain compound **1e**.

¹H NMR(400MHz, CDCl₃) δ ppm 8.08 (s, 1H), 4.04-4.23 (m, 4H), 3.27 (s, 3H), 1.95-2.06 (m, 2H), 1.77-1.88 (m, 2H).

### Step 5

Compound **1e** (76.1 mg, 300 µmol, 1 *eq*), compound **If** (53.3 mg, 360 µmol, 1.2 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II)(54.4 mg, 60 µmol, 0.2 *eq)* and cesium carbonate (146.6 mg, 450 µmol, 1.5 *eq)* were placed in a reaction flask, and the system was replaced with nitrogen for three times, then 10 mL of anhydrous dioxane was added to the mixture and reacted at 100 °C for 8 hours. After the reaction was completed, the reaction solution was filtered through diatomite, and the filtrate was concentrated under reduced pressure to obtain a crude product, and purified by preparative high performance liquid chromatography (Welch Xtimate C18 150^{∗}30 mm^{∗}5 µm; mobile phase: [water (0.225 % formic acid)-acetonitrile]; acetonitrile %: 13 %-43 %, 8 minutes) to obtain compound **1**. MS: m/z 366.2 [M+H]⁺.

¹H NMR (400 MHz, DMSO-*d*₆) δ ppm 9.14 (s, 1H), 8.86 (s, 1H), 8.38 (s, 1H), 8.16 (s, 1H), 7.72 (s, 1H), 3.83-4.05 (m, 4H), 3.15 (s, 3H), 2.39 (s, 3H), 1.77-1.88 (m, 2H), 1.63-1.73 (m, 2H).

### Embodiment 2

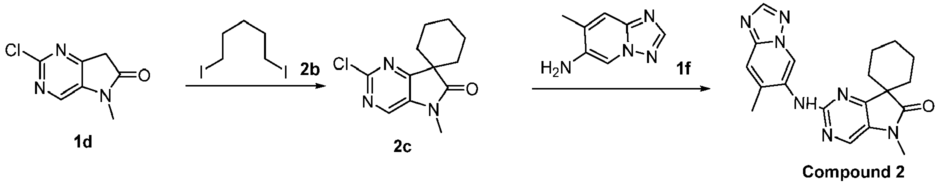

### Step 1

**2b** (518.31 mg, 1.6 mmol, 2 *eq*) and cesium carbonate (1.04 g, 3.2 mmol, 4 *eq*) were sequentially added to a *N,N*-dimethylformamide (10 mL) solution of compound **Id** (146.8 mg, 0.8 mmol, 1 *eq);* and after the addition was completed, the reaction solution was reacted at 60 °C for 12 hours. After the reaction was completed, the reaction solution was diluted with 30 mL of water, extracted with ethyl acetate (20 mL^{∗}3), washed with 20 mL of saturated brine, dried with anhydrous sodium sulfate, filtered, and the filtrate was concentrated under reduced pressure and purified by silica gel column chromatography (ethyl acetate: petroleum ether= 1: 2) to obtain compound **2c**.

¹H NMR(400 MHz, CDCl₃) δ ppm 8.03 (s, 1H), 3.24 (s, 3H), 1.85-2.02 (m, 4H), 1.70-1.82 (m, 4H), 1.56-1.66 (m, 2H).

### Step 2

Compound **2c** (100 mg, 397.28 µmol, 1 *eq*), compound **If** (70.6 mg, 476.74 µmol, 1.2 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (72.0 mg, 79.46 µmol, 0.2 *eq*) and cesium carbonate (258.9 mg, 794.56 µmol, 2 *eq*) were placed in a reaction flask, and the system was replaced with nitrogen for three times, then 15 mL of anhydrous dioxane was added to the mixture and reacted at 100 °C for 12 hours. After the reaction was completed, the reaction solution was filtered through diatomite, and the filtrate was concentrated under reduced pressure to obtain a crude product, and purified by preparative high performance liquid chromatography (Welch Xtimate C18 150^{∗}30 mm^{∗}5 µm; mobile phase: [water (0.225 % formic acid)-acetonitrile]; acetonitrile %: 33 %-53 %, 8 minutes) to obtain compound **2**. MS: m/z: 364.2 [M+H]⁺.

¹H NMR (400 MHz, CDCl₃) δ ppm 9.80 (s, 1H), 8.26 (s, 1H), 7.92 (s, 1H), 7.58 (s, 1H), 6.81 (s, 1H), 3.24 (s, 3H), 2.53 (s, 3H), 1.98-2.08 (m, 2H), 1.90-1.95 (m, 2H), 1.82-1.87 (m, 2H), 1.72-1.81 (m, 2H), 1.64-1.72 (m, 2H).

### Embodiments 3, 4

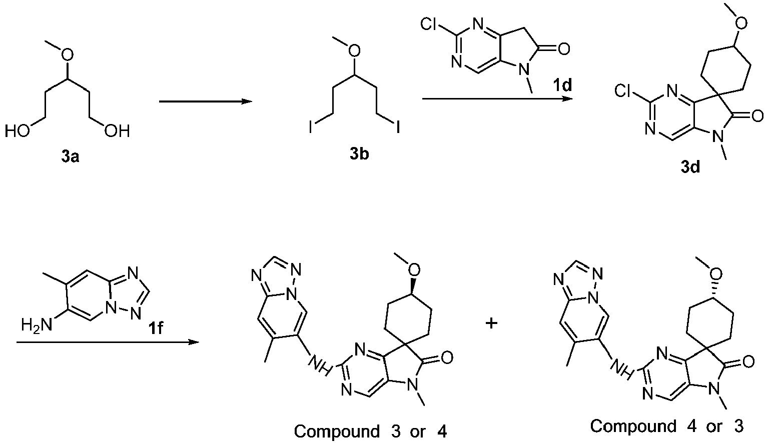

### Step 1

At 0 °C, imidazole (0.926 g, 13.61 mmol, 2.2 *eq*), triphenylphosphine (3.25 g, 12.37 mmol, 2 *eq*) and iodine (3.14g, 12.37mmol, 2 *eq)* were sequentially added to a tetrahydrofuran (30 mL) solution of compound **3a** (0.83 g, 6.19 mmol, 1 *eq),* and the reaction solution was first reacted at 0 °C for 1 hour, and then the reaction solution was reacted at 15 °C for 5 hours. After the reaction was completed, the reaction solution was quenched with 20 mL of saturated sodium thiosulfate solution, extracted with ethyl acetate (50 mL^{∗}3), washed with 30 mL of saturated brine, dried with anhydrous sodium sulfate, filtered, and the filtrate was concentrated under reduced pressure and purified by silica gel column chromatography (ethyl acetate: petroleum ether= 1: 4) to obtain compound **3b**.

¹H NMR (400 MHz, CDCl₃) δ ppm 3.42 (s, 3H), 3.34-3.39 (m, 1H), 3.17-3.29 (m, 4H), 1.94-2.12 (m, 4H).

### Step 2

Cesium carbonate (0.977 g, 3 mmol, 4 *eq*) and compound **3b** (0.796 g, 4 mmol, 3 *eq*) were sequentially added to a *N,N*-dimethylformamide (25 mL) solution of compound **Id** (0.138 g, 0.75 mmol, 1 *eq);* and after the addition was completed, the reaction solution was reacted at 50 °C for 6 hours. After the reaction was completed, the reaction solution was diluted with 50 mL of water, extracted with ethyl acetate (50 mL^{∗}3), washed with 30 mL of saturated brine, dried with anhydrous sodium sulfate, filtered, and the filtrate was concentrated under reduced pressure and purified by silica gel column chromatography (ethyl acetate: petroleum ether= 1: 3) to obtain compound **3d**. MS: *m*/*z* 281.8 [M+H]⁺.

### Step 3

Compound **3d** (140.9 mg, 500 µmol, 1 *eq*), compound **If** (74.1 mg, 500 µmol, 1 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (90.7 mg, 100 µmol, 0.2 *eq)* and cesium carbonate (244.4 mg, 750 µmol, 1.5 *eq*) were placed in a reaction flask, and the system was replaced with nitrogen for three times, then 20 mL of anhydrous dioxane was added to the mixture and reacted at 100 °C for 8 hours. After the reaction was completed, the reaction solution was filtered through diatomite, and the filtrate was concentrated under reduced pressure to obtain a crude product, and purified by preparative high performance liquid chromatography (Welch Xtimate C18 150^{∗}30 mm^{∗}5 µm; mobile phase: [water (0.225 % formic acid)-acetonitrile]; acetonitrile %: 25 %-45 %, 8 minutes) to obtain compound **3** (Ultimate XB-C18 3.0^{∗}50 mm, 3 µm; acetonitrile %: 0 %-60 % , 10 minutes; retention time was 3.86 min), compound **4** (Ultimate XB-C18 3.0^{∗}50 mm, 3 µm; acetonitrile %: 0 %-60 %, 10 minutes; retention time was 3.93 min).

### Compound 3:

MS: m/z 394.2 [M+H]⁺.

¹H NMR (400 MHz, DMSO-*d*₆) δ ppm 9.06 (s, 1H), 8.84 (s, 1H), 8.37 (s, 1H), 8.09 (s, 1H), 7.70 (s, 1H), 3.22 (s, 3H), 3.12 (s, 3H), 2.37 (s, 3H), 1.88-2.06 (m, 4H), 1.77-1.86 (m, 2H), 1.58-1.70 (m, 2H).

### Compound 4:

MS: m/z 394.4 [M+H]⁺.

¹H NMR (400 MHz, DMSO-*d*₆) δ ppm 9.13 (s, 1H), 8.79 (s, 1H), 8.37 (s, 1H), 8.12 (s, 1H), 7.70 (s, 1H), 3.26 (s, 3H), 3.12 (s, 3H), 2.38 (s, 3H), 2.12-2.14 (m, 2H), 1.81-1.97 (m, 4H), 1.44-1.54 (m, 2H).

### Embodiment 5

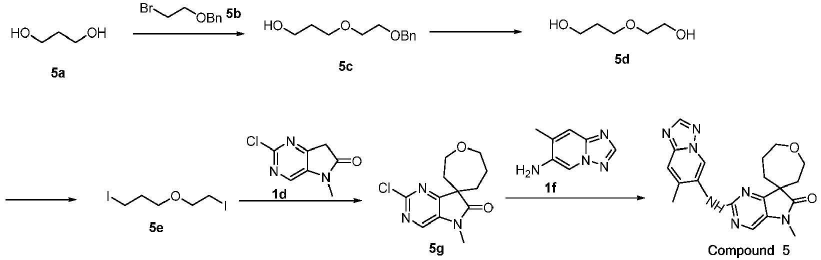

### Step 1

Sodium hydride (6.0 g, 150 mmol, 60 % purity, 1.5 *eq*) was added to a *N,N-*dimethylformamide (120 mL) solution of compound **5a** (21.51 g, 100 mmol, 1 *eq*) at 0 °C, and the mixture was stirred at 0 °C for 0.5 hours, then compound **5b** (9.13 g, 120 mmol, 1.2 *eq)* was added; after the addition was completed, the reaction solution was reacted at 15 °C for 11.5 hours. After the reaction was completed, the reaction solution was quenched with 100 mL of water at 0 °C, extracted with ethyl acetate (150 mL^{∗}3), washed with 50 mL of saturated brine, dried with anhydrous sodium sulfate, filtered, and the filtrate was concentrated under reduced pressure and purified by silica gel column chromatography (ethyl acetate: petroleum ether= 1: 1) to obtain compound **5c**.

¹H NMR (400 MHz, CDCl₃) δ ppm 7.18-7.29 (m, 5H), 4.49 (s, 2H), 3.71 (t, *J*=5.52 Hz, 2H), 3.53-3.64 (m, 6H), 2.46 (br s, 1H), 1.77 (quin, *J*=5.71 Hz, 2H).

### Step 2

An ethanol (80 mL) solution of compound **5c** (8.41 g, 40 mmol, 1 *eq*) was replaced with nitrogen for three times, and Pd/C (1 g, 10% purity) was added; under an atmospheric pressure of hydrogen, the reaction was reacted at 15 °C for 4 hours and then reacted at 70 °C for 4 hours. After the reaction was completed, the reaction solution was filtered through diatomite, and the filtrate was concentrated under reduced pressure to obtain compound **5d**.

¹H NMR (400 MHz, CDCl₃) δ ppm 3.77 (t, *J*=5.65 Hz, 2H), 3.70-3.74 (m, 2H), 3.66 (t, *J*=5.77 Hz, 2H), 3.54-3.60 (m, 2H), 2.50-2.83 (m, 2H), 1.79-1.89 (m, 2H).

### Step 3

At 0 °C, imidazole (3.0 g, 44 mmol, 2.2 *eq*), triphenylphosphine (10.49 g, 40 mmol, 2 *eq*) and iodine (10.15 g, 40 mmol, 2 *eq*) were sequentially added to a tetrahydrofuran (80 mL) solution of compound **5d** (2.4 g, 20 mmol, 1 *eq),* and the reaction solution was first reacted at 0 °C for 1 hour, and then reacted at 15 °C for 3 hours. After the reaction was completed, the reaction solution was quenched with 20 mL of saturated sodium thiosulfate solution, extracted with ethyl acetate (50 mL^{∗}3), washed with 50 mL of saturated brine, dried with anhydrous sodium sulfate, filtered, and the filtrate was concentrated under reduced pressure and purified by silica gel column chromatography (ethyl acetate: petroleum ether= 1: 9) to obtain compound **5e**.

¹H NMR (400 MHz, CDCl₃) δ ppm 3.70 (t, *J*=6.65 Hz, 2H), 3.55 (t, *J*=5.77 Hz, 2H), 3.28-3.34 (m, 2H), 3.21-3.27 (m, 2H), 2.05 (quin, *J*=6.21 Hz, 2H).

### Step 4

Cesium carbonate (1.43 g, 4.4 mmol, 4 *eq)* and compound **5e** (1.12 g, 3.3 mmol, 3 *eq)* were sequentially added to a *N,N*-dimethylformamide (25 mL) solution of compound **Id** (0.202 g, 1.1 mmol, 1 *eq);* and after the addition was completed, the reaction solution was reacted at 50 °C for 6 hours. After the reaction was completed, the reaction solution was diluted with 50 mL of water, extracted with ethyl acetate (50 mL^{∗}3), washed with 30 mL of saturated brine, dried with anhydrous sodium sulfate, filtered, and the filtrate was concentrated under reduced pressure and purified by silica gel column chromatography (ethyl acetate: petroleum ether= 1: 2) to obtain compound **5g**. MS: *m*/*z* 267.8 [M+H]⁺.

### Step 5

Compound **5g** (53.5 mg, 200 µmol, 1 *eq*), compound **If** (35.6 mg, 240 µmol, 1.2 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (36.3 mg, 40 µmol, 0.2 *eq)* and cesium carbonate (97.8 mg, 300 µmol, 1.5 *eq*) were placed in a reaction flask, and the system was replaced with nitrogen for three times, then 8 mL of anhydrous dioxane was added to the mixture and reacted at 100 °C for 8 hours. After the reaction was completed, the reaction solution was filtered through diatomite, and the filtrate was concentrated under reduced pressure to obtain a crude product, and purified by preparative high performance liquid chromatography (Welch Xtimate C18 150^{∗}30 mm^{∗}5 µm; mobile phase: [water (0.225 % formic acid)-acetonitrile]; acetonitrile %: 17 %-37 %, 8 minutes) to obtain compound **5**. MS: *m*/*z* 380.3 [M+H]⁺.

¹H NMR (400 MHz, CDCl₃) δ ppm 9.79 (s, 1H), 8.26 (s, 1H), 7.93 (s, 1H), 7.59 (s, 1H), 6.90 (s, 1H), 4.04 (t, *J*=4.64 Hz, 2H), 3.80-3.99 (m, 2H), 3.24 (s, 3H), 2.53 (s, 3H), 2.00-2.16 (m, 6H).

### Embodiment 6

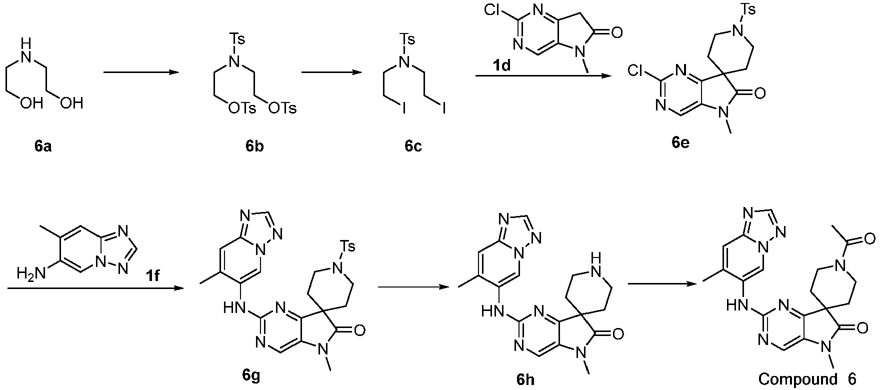

### Step 1

Triethylamine (115.50 g, 1.14 mol, 158.87 mL, 6 *eq*) and *p*-toluenesulfonyl chloride (362.67 g, 1.90 mol, 10 *eq*) were sequentially added to an anhydrous dichloromethane (200 mL) solution of compound **6a** (20 g, 190.23 mmol, 18.35 mL, 1 *eq)* at 0 °C; and after the addition was completed, the reaction solution was reacted at 25 °C for 60 hours. After the reaction was completed, the mixture was concentrated under reduced pressure to obtain a crude product, and purified by silica gel column chromatography (ethyl acetate: petroleum ether= 1: 1) to obtain compound **6b**. MS: *m*/*z.* 568.0 [M+H]⁺.

¹H NMR (400MHz, DMSO-*d*₆) δ ppm 7.74 (d, *J*=8.3 Hz, 4H), 7.58 (d, *J*=8.3 Hz, 2H), 7.49 (d, *J*=8.0 Hz, 4H), 7.37 (d, *J*=8.3 Hz, 2H), 4.01 (t, *J*=5.9 Hz, 4H), 3.30 (t, *J*=5.9 Hz, 4H), 2.44 (s, 6H), 2.39 (s, 3H).

### Step 2

Sodium iodide (13.20 g, 88.08 mmol, 5 *eq*) was added to an acetone (100 mL) solution of compound **6b** (10 g, 17.62 mmol, 1 *eq*), and the reaction solution was reacted at 70 °C for 20 hours. After the reaction was completed, the mixture was concentrated under reduced pressure to obtain a crude product, and purified by silica gel column chromatography (ethyl acetate: petroleum ether= 1: 4) to obtain compound **6c**. MS: *m*/*z* 479.7 [M+H]⁺.

### Step 3

Cesium carbonate (2.45 g, 7.53 mmol, 4 *eq*) and compound **6c** (2.70 g, 5.65 mmol, 3 *eq*) were added to a *N,N*-dimethylformamide (75 mL) solution of compound **1d** (345.5 mg, 1.88 mmol, 1 *eq*), and the reaction solution was reacted at 50 °C for 15 hours. After the reaction was completed, the mixture was concentrated under reduced pressure to obtained a crude product, and purified by silica gel column chromatography (ethyl acetate: petroleum ether= 4: 1) to obtain compound **6e**. MS: *m*/*z* 407.0 [M+H]⁺.

### Step 4

Compound **6e** (265.9 mg, 653.50 µmol, 1 *eq*), compound **If** (116.2 mg, 784.20 µmol, *1.2 eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (118.5 mg, 130.70 µmol, 0.2 *eq*) and cesium carbonate (425.9 mg, 1.31 mmol, 2 *eq)* were placed in a reaction flask, and the system was replaced with nitrogen for three times, then 52 mL of anhydrous dioxane was added to the mixture and reacted at 100 °C for 20 hours. After the reaction was completed, the mixture was concentrated under reduced pressure to obtained a crude product, and purified by silica gel column chromatography (pure ethyl acetate) to obtain compound **6g**. MS: m/z 519.1 [M+H]⁺.

### Step 5

Compound **6g** (89.3 mg, 172.20 µmol, 1 *eq)* was dissolved in an acetic acid (5 mL, 33 %) solution of hydrogen bromide, then phenol (105.3 mg, 1.12 mmol, 98.45 µL, 6.5 *eq*) was added dropwise, and the reaction solution was reacted for 5.5 hours at 30 °C. After the reaction was completed, the mixture was concentrated under reduced pressure to obtained a crude product, and purified by preparative high performance liquid chromatography (Welch Xtimate C18 150^{∗}30 mm^{∗}5 µm; mobile phase: water (0.225 % formic acid)-acetonitrile]; acetonitrile %: 0 %-30 %, 8 minutes) to obtain compound **6h**. MS: *m*/*z* 365.2 [M+H]⁺.

### Step 6

Triethylamine (13.3 mg, 131.72 µmol, 18.33 µL, 3 *eq)* and acetic anhydride (5.4 mg, 52.69 µmol, 4.93 µL, 1.2 *eq*) were sequentially added to an anhydrous tetrahydrofuran (2 mL) solution of compound **6h** (16 mg, 43.91 µmol, 1 *eq*) at 0 °C, then the reaction solution was stirred at 30 °C for 2 hours. After the reaction was completed, the mixture was concentrated under reduced pressure to obtained a crude product, and purified by preparative high performance liquid chromatography (Welch Xtimate C18 150^{∗}30 mm^{∗}5 µm; mobile phase: [water (0.225 % formic acid)-acetonitrile]; acetonitrile %: 18 %-28 %, 8 minutes) to obtain compound **6**. MS: m/z 407.2 [M+H]⁺.

¹H NMR (400MHz, DMSO-*d*₆) δ ppm 9.15 (s, 1H), 8.86 (s, 1H), 8.38 (s, 1H), 8.17 (s, 1H), 7.72 (s, 1H), 4.06-3.60 (m, 4H), 3.16 (s, 3H), 2.39 (s, 3H), 2.05 (s, 3H), 1.87-1.66 (m, 4H).

### Embodiment 7

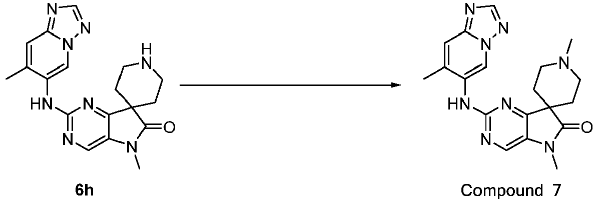

Acetic acid (49.4 mg, 823.26 µmol, 47.08 µL, 3 *eq*) and paraformaldehyde (41.2 mg, 1.37 mmol, 5 *eq*) were added to an anhydrous methanol (4 mL) solution of compound **6h** (100 mg, 274.42 µmol, 1 *eq*), and the reaction solution was reacted at 25 °C for 1 hour, then sodium cyanoborohydride (34.5 mg, 548.84 µmol, 2 *eq*) was added to the reaction system, and the reaction solution was reacted at 25 °C for 17 hours. After the reaction was completed, the reaction solution was quenced with saturated sodium bicarbonate solution (5 mL), concentrated under reduced pressure to obtained a crude product, and purified by preparative high performance liquid chromatography (Phenomenex Gemini-NX 80^{∗}30 mm^{∗}3 µm; mobile phase: [water (10 mM sodium bicarbonate)-acetonitrile]; acetonitrile %: 15 %-25 %, 9.5 minutes) to obtain compound 7. MS: *m*/*z* 379.2 [M+H]⁺;

¹H NMR (400MHz, CDCl₃) δ ppm 9.75 (br s, 1H), 8.27 (s, 1H), 7.95 (s, 1H), 7.59 (s, 1H), 6.85 (s, 1H), 3.25 (s, 3H), 2.88 (s, 4H), 2.51 (d, *J*=18.4 Hz, 6H), 2.05 (s, 2H), 1.94 (s, 2H).

### Embodiment 8

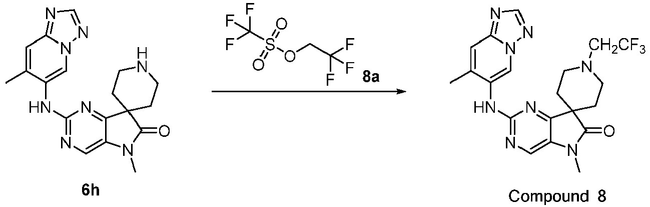

Compound **8a** (76.4 mg, 329.31 µmol, 47.47 µL, 1.2 *eq*) and triethylamine (55.5 mg, 548.84 µmol, 76.39 µL, 2 *eq)* were added to an acetonitrile (5 mL) solution of compound **6h** (100 mg, 274.42 µmol, 1 *eq);* and after the addition was completed, the reaction solution was reacted at 25 °C for 6 hours. After the reaction was completed, the mixture was concentrated under reduced pressure to obtained a crude product, and purified by thin layer chromatography (dichloromethane: methanol= 20: 1) to obtain compound **8**. MS: *m*/*z* 447.2 [M+H]⁺;

¹H NMR (400MHz, CDCl₃) δ ppm 9.77 (s, 1H), 8.26 (s, 1H), 7.95 (s, 1H), 7.58 (s, 1H), 6.81 (s, 1H), 3.25 (s, 3H), 3.04-3.22 (m, 6H), 2.52 (s, 3H), 2.06 (ddd, *J*=4.0, 8.7, 13.2 Hz, 2H), 1.93-1.83 (m, 2H).

### Embodiments 9, 10

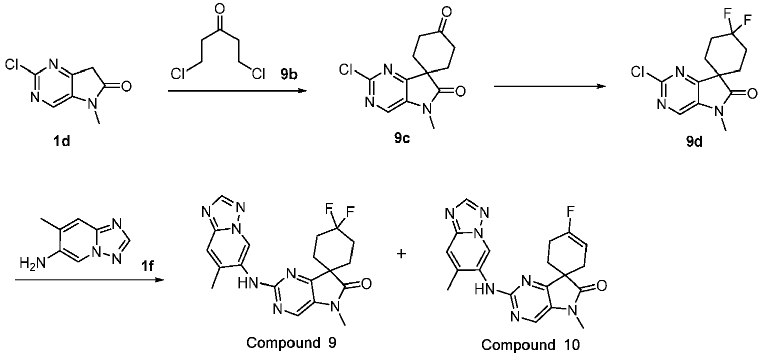

### Step 1

Cesium carbonate (0.717 g, 2.2 mmol, 4 *eq*), sodium iodide (82.4 mg, 0.55 mmol, 1 *eq*) and compound **9b** (0.256 g, 1.65 mmol, 3 *eq)* were sequentially added to a *N,N-*dimethylformamide (8 mL) solution of compound **Id** (0.101 g, 0.55 mmol, 1 *eq);* and after the addition was completed, the reaction solution was reacted at 50 °C for 12 hours. After the reaction was completed, the reaction solution was diluted with 20 mL of water, extracted with ethyl acetate (30 mL^{∗}3), washed with 20 mL of saturated brine, dried with anhydrous sodium sulfate, filtered, and the filtrate was concentrated under reduced pressure and purified by silica gel column chromatography (ethyl acetate: petroleum ether= 1: 1) to obtain compound **9c**. MS: *m*/*z.* 266.0 [M+H]⁺.

¹H NMR (400 MHz, CDCl₃) δ ppm 8.14 (s, 1H), 3.31 (s, 3H), 2.89-2.94 (m, 2H) 2.75-2.82 (m, 2H) 2.23-2.32 (m, 2H) 2.12-2.21 (m, 2H).

### Step 2

Diethylaminosulfur trifluoride (0.290 g, 1.8 mmol, 3 *eq)* was added to a dichloromethane (10 mL) solution of compound **9c** (159.4 mg, 0.6 mmol, 1 *eq);* and after the addition was completed, the reaction solution was reacted at 20 °C for 12 hours. After the reaction was completed, the reaction solution was quenched with 30 mL of saturated sodium bicarbonate solution, extracted with ethyl acetate (30 mL^{∗}3), washed with 10 mL of saturated brine, dried with anhydrous sodium sulfate, filtered, and the filtrate was concentrated under reduced pressure and purified by silica gel column chromatography (ethyl acetate: petroleum ether= 1: 1.5) to obtain compound **9d**. MS: *m*/*z* 287.9 [M+H]⁺.

### Step 3

Compound **9d** (77.7 mg, 270 µmol, 1 *eq*), compound **If** (48.6 mg, 324 µmol, 1.2 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (49.0 mg, 54 µmol, 0.2 *eq*) and cesium carbonate (132 mg, 405 µmol, 1.5 *eq)* were placed in a reaction flask, and the system was replaced with nitrogen for three times, then 10 mL of anhydrous dioxane was added to the mixture and reacted at 100 °C for 8 hours. After the reaction was completed, the reaction solution was filtered through diatomite, and the filtrate was concentrated under reduced pressure to obtained a crude product, and purified by preparative high performance liquid chromatography (Phenomenex Gemini-NX 80^{∗}30 mm^{∗}3 µm; mobile phase: [water (10 mM sodium bicarbonate)-acetonitrile]; acetonitrile %: 30 %-60 %, 9.5 minutes) to obtain compound **9** and compound **10**.

### Compound 9:

MS: *m*/*z* 400.1 [M+H]⁺.

¹H NMR (400 MHz, CDCl₃) δ ppm 9.72 (s, 1H), 8.27 (s, 1H), 7.96 (s, 1H), 7.59 (s, 1H), 6.80 (s, 1H), 3.26 (s, 3H), 2.53 (s, 3H), 2.35-2.48 (m, 4H), 2.06-2.15 (m, 2H), 1.93-2.01 (m, 2H).

### Compound 10:

MS: *m*/*z* 380.1 [M+H]⁺.

¹H NMR (400 MHz, CDCl₃) δ ppm 9.71 (s, 1H), 8.26 (s, 1H), 7.97 (s, 1H), 7.58 (s, 1H), 6.88 (s, 1H), 5.31-5.38 (m, 1H), 3.27 (s, 3H), 2.60-2.73 (m, 2H), 2.46-2.54 (m, 4H), 2.13-2.31 (m, 2H), 1.89-1.96 (m, 1H).

### Embodiment 11

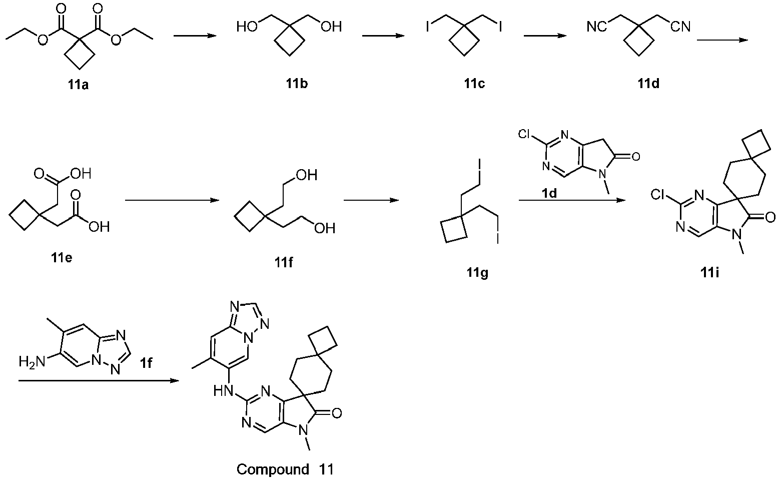

### Step 1

Lithium aluminum tetrahydride (5.69 g, 149.83 mmol, 3 *eq*) was added to an anhydrous tetrahydrofuran (200 mL) solution of compound **11a** (10 g, 49.94 mmol, 9.52 mL, 1 *eq*) at 0 °C; after the addition was completed, the reaction solution was transferred to 30 °C and reacted for 3 hours. After the reaction was completed, tetrahydrofuran (200 mL) was added to dilute, and the mixture was cooled to 0 °C, and water (5.7 mL), 20 % sodium hydroxide solution (5.7 mL) and water (17 mL) were sequentially added to the reaction solution, and then the mixture was stirred at room temperature for 30 minutes, filtered, and the filtrate was concentrated under reduced pressure to obtain crude compound **11b**.

¹H NMR (400 MHz, CDCl₃) δ ppm 3.73 (d, *J*=5.2 Hz, 4H), 2.62 (t, *J*=5.2 Hz, 2H), 1.90-2.10 (m, 2H), 1.70-1.80 (m, 4H).

### Step 2

At 0 °C, iodine (59.43 g, 234.16 mmol, 47.17 mL, 4 *eq*) was added to a dichloromethane (300 mL) solution of imidazole (31.88 g, 468.33 mmol, 8 *eq)* and triphenylphosphine (61.42 g, 234.16 mmol, 4 *eq).* After the addition was completed, the reaction solution was reacted at 0 °C for 1 hour, then a dichloromethane (10 mL) solution of compound **11b** (6.8 g, 58.54 mmol, 1 *eq*) was added. After the addition was completed, the reaction solution was transferred to 30 °C and reacted for 2 hours. After the reaction was completed, the reaction solution was diluted with water (300 mL), extracted with dichloromethane (300 mL^{∗}2), dried with anhydrous sodium sulfate, filtered, and the filtrate was concentrated under reduced pressure and purified by silica gel column chromatography (pure petroleum ether) to obtain compound **11c**.

¹H NMR (400 MHz, CDCl₃) δ ppm 3.53 (s, 4H), 1.90-2.00 (m, 4H), 1.75-1.85 (m, 2H).

### Step 3

Potassium cyanide (4.15 g, 63.73 mmol, 2.73 mL, 4.28 *eq*) was added to a *N,N-*dimethylformamide (30 mL) solution of compound **11c** (5 g, 14.88 mmol, 1 *eq);* after the addition was completed, the reaction solution was reacted at 80 °C for 16 hours. After the reaction was completed, the reaction solution was cooled to room temperature, diluted with water (100 mL), extracted with ethyl acetate (80 mL^{∗}3), washed sequentially with water (80 mL^{∗}3) and saturated brine (80 mL^{∗}2), dried with anhydrous sodium sulfate, filtered, and the filtrate was concentrated under reduced pressure to obtain crude compound **11d**.

¹H NMR (400 MHz, CDCl₃) δ ppm 2.65 (s, 4H), 2.10-2.20 (m, 4H), 2.00-2.10 (m, 2H).

### Step 4

Concentrated hydrochloric acid (10 mL) was added to compound **11d** (2 g, 14.91 mmol, 1 *eq);* after the addition was completed, and the reaction solution was reacted at 100 °C for 16 hours. After the reaction was completed, the reaction solution was cooled to 30 °C, filtered, and the filter cake was dried to obtain compound **11e**.

¹H NMR (400 MHz, DMSO-*d*₆) δ ppm 12.04 (br s, 2H), 2.55-2.60 (m, 4H), 1.90-2.00 (m, 4H), 1.80-1.90 (m, 2H).

### Step 5

Lithium aluminum tetrahydride (1.59 g, 41.82 mmol, 4 *eq*) was added to an anhydrous tetrahydrofuran (200 mL) solution of compound **11e** (1.8 g, 10.45 mmol, 9.52 mL, 1 *eq*) at 0 °C; after the addition was completed, the reaction solution was transferred to 30 °C and reacted for 3 hours. After the reaction was completed, tetrahydrofuran (200 mL) was added to dilute. The reaction solution was cooled to 0 °C, and water (1.6 mL), 20 % sodium hydroxide solution (1.6 mL) and water (5 mL) were sequentially added, and then the mixture was stirred at room temperature for 30 minutes, filtered, and the filtrate was concentrated under reduced pressure to obtain crude compound **11f**.

¹H NMR (400 MHz, DMSO-d₆): δ ppm 3.65-3.75 (m, 4H), 1.95-2.05 (m, 2H), 1.85-1.95 (m, 2H), 1.75-1.85 (m, 8H).

### Step 6

At 0 °C, iodine (9.86 g, 38.83 mmol, 7.82 mL, 4 *eq*) was added to a dichloromethane (50 mL) solution of imidazole (5.29 g, 77.66 mmol, 8 *eq*) and triphenylphosphine (10.19 g, 38.83 mmol, 4 *eq).* After the addition was completed, the reaction solution was reacted at 0 °C for 1 hour. Then compound **11f** (1.4 g, 9.71 mmol, 1 *eq*) was added to the reaction solution, after the addition was completed, the reaction solution was transferred to 30 °C and reacted for 2 hours. After the reaction was completed, the reaction solution was diluted with 40 mL of water, extracted with dichloromethane (20 mL^{∗}2), dried with anhydrous sodium sulfate, filtered, and the filtrate was concentrated under reduced pressure and purified by silica gel column chromatography (pure petroleum ether) to obtain compound **11g**.

¹H NMR (400 MHz, CDCl₃): δ ppm 3.00-3.10 (m, 4H), 2.10-2.20 (m, 4H), 1.85-1.95 (m, 2H), 1.75-1.85 (m, 4H).

### Step 7

Compound **11g** (594.8 mg, 1.63 mmol, 2 *eq)* and cesium carbonate (532.4 mg, 1.63 mmol, 2 *eq)* were sequentially added to a *N,N*-dimethylformamide (2 mL) solution of compound **1d** (150 mg, 817.02 µmol, 1 *eq*); and after the addition was completed, the reaction solution was reacted at 100 °C for 16 hours. After the reaction was completed, the reaction solution was cooled to 30 °C, diluted with water (30mL), extracted with ethyl acetate (50 mL^{∗}3), washed sequentially with water (50 mL^{∗}3) and saturated brine (30 mL^{∗}2), dried with anhydrous sodium sulfate, filtered, and the filtrate was concentrated under reduced pressure to obtain a crude product, then the crude product was purified by preparative thin layer chromatography (methanol: dichloromethane=1: 10) to obtain compound **11i**.

MS: *m*/*z.* 291.9 [M+H]⁺.

¹H NMR (400 MHz, CDCl₃): δ ppm 8.01 (s, 1H), 3.23 (s, 3H), 2.00-2.10 (m, 2H), 1.90-2.00 (m, 8H), 1.75-1.85 (m, 2H), 1.60-1.70 (m, 2H).

### Step 8

Compound **If** (48.8 mg, 329.02 µmol, 1.2 *eq*), cesium carbonate (134.0 mg, 411.28 µmol, 1.5 *eq)* and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (49.7 mg, 54.84 µmol, 0.2 *eq*) were sequentially added to a dioxane (2 mL) solution of compound **11i** (80 mg, 274.18 µmol, 1 *eq);* after the addition was completed, the reaction solution was replaced with nitrogen for three times, then reacted at 100 °C for 3 hours under the protection of nitrogen. After the reaction was completed, the mixture was cooled to 30 °C, diluted with ethyl acetate (20 mL), filtered through diatomite, washed with ethyl acetate, and the obtained filtrate was concentrated under reduced pressure to obtain a crude product, and purified by preparative thin layer chromatography (pure ethyl acetate) to obtain compound **11**. MS: m/z 404.3 [M+H]⁺.

¹H NMR (400 MHz, CDCl₃): δ ppm 9.72 (s, 1H), 8.25 (s, 1H), 7.91 (s, 1H), 7.57 (s, 1H), 6.80 (s, 1H), 3.23 (s, 3H), 2.52 (s, 3H), 2.00-2.10 (m, 2H), 1.80-2.00 (m, 6H), 1.60-1.80 (m, 6H).

### Embodiment 12

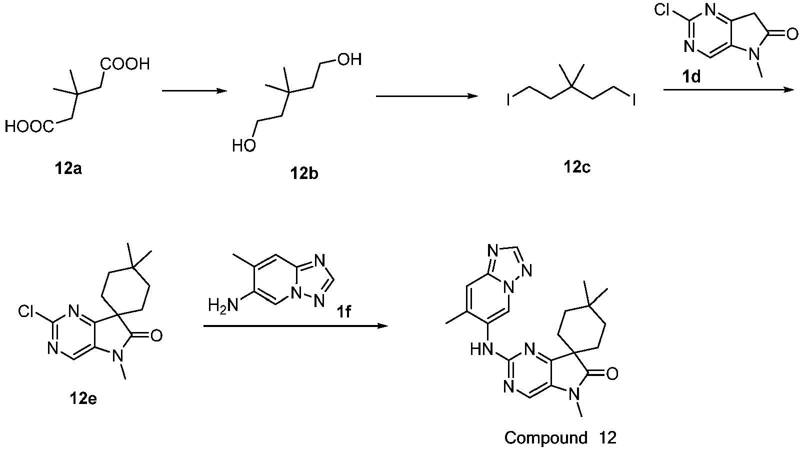

### Step 1

At 0 °C, lithium aluminum tetrahydride (2.37 g, 62.43 mmol, 2 *eq*) was added to an anhydrous tetrahydrofuran (50 mL) solution; and compound **12a** (5 g, 31.22 mmol, 1 *eq*) was dissolved in an anhydrous tetrahydrofuran (25 mL) solution, and slowly added dropwise to the reaction system; after the addition was completed, the reaction solution was reacted at 25 °C for 3.5 hours and then reacted at 80 °C for 13 hours. After the reaction was completed, the reaction solution was cooled to room temperature, then water (2.4 mL) and 15 % aqueous sodium hydroxide solution (2.4 mL) were added to the reaction system and stirred for 15 minutes, and then water (7.2 mL) was added and stirred continuously for 15 minutes. After the quenching was completed, anhydrous sodium sulfate was added to dry, and the mixture was filtered, and the filtrate was concentrated under reduced pressure to obtain compound **12b**.

¹H NMR (400MHz, CDCl₃) δ ppm 3.75 (t, *J*=7.2 Hz, 4H), 1.60 (t, *J*=7.2 Hz, 4H), 1.36-1.20 (m, 2H), 0.97 (s, 6H).

### Step 2

At 0 °C, compound **12b** (4.58 g, 34.64 mmol, 1 *eq)* was slowly added to a mixed anhydrous dichloromethane (180 mL) solution of imidazole (18.87 g, 277.16 mmol, 8 *eq*), triphenylphosphine (36.35 g, 138.58 mmol, 4 *eq*) and iodine (35.17 g, 138.58 mmol, 27.91 mL, *4 eq*), and the reaction solution was first reacted at 0 °C for 1 hour, and then reacted at 30 °C for 3 hours. After the reaction was completed, the reaction solution was quenched with saturated sodium thiosulfate solution (20 mL), extracted with 150 mL of ethyl acetate (50 mL^{∗}3), dried with anhydrous sodium sulfate, filtered, and the filtrate was concentrated under reduced pressure and purified by silica gel column chromatography (pure petroleum ether) to obtain compound **12c**.

¹H NMR (400MHz, CDCl₃) δ ppm 3.18-3.11 (m, 4H), 1.99-1.87 (m, 4H), 0.92 (s, 6H).

### Step 3

Cesium carbonate (1.77 g, 5.45 mmol, 5 *eq)* and compound **12c** (1.15 g, 3.27 mmol, *3 eq*) were added to a *N,N*-dimethylformamide (33 mL) solution of compound **Id** (200 mg, 1.09 mmol, 1 *eq),* and the reaction solution was reacted at 100 °C for 9 hours. After the reaction was completed, the reaction solution was concentrated under reduced pressure, diluted with water (10 mL), extracted with ethyl acetate (30 mL^{∗}3), dried with anhydrous sodium sulfate, filtered, and the filtrate was concentrated under reduced pressure and purified by column chromatography (ethyl acetate: petroleum ether= 1: 2) to obtain compound **12e**. MS: m/z 279.9 [M+H]⁺.

### Step 4

Compound **12e** (136 mg, 486.12 µmol, 1 *eq*), compound **1f** (64.8 mg, 437.51 µmol, 0.9 *eq),* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (88.1 mg, 97.22 µmol, 0.2 *eq)* and cesium carbonate (316.8 mg, 972.25 µmol, 2 *eq)* were placed in a reaction flask, and the system was replaced with nitrogen for three times, then 3 mL of anhydrous dioxane was added to the mixture and reacted at 100 °C for 16 hours. After the reaction was completed, the reaction solution was filtered through diatomite, and the filtrate was concentrated under reduced pressure to obtain a crude product, and purified by preparative high performance liquid chromatography (Phenomenex Gemini-NX 80^{∗}30 mm^{∗}3 µm; mobile phase: [water (10 mM ammonium bicarbonate)-acetonitrile]; acetonitrile %: 33 %-63 %, 9.5 minutes) to obtain compound 12. MS: m/z 392.3 [M+H]⁺.

¹H NMR (400MHz, CDCl₃) δ ppm 9.71 (s, 1H), 8.26 (s, 1H), 7.93 (s, 1H), 7.59 (br s, 1H), 6.79 (br s, 1H), 3.25 (s, 3H), 2.53 (s, 3H), 1.95 (br d, *J*=9.0 Hz, 2H), 1.83 (br s, 2H), 1.68 (br s, 4H), 1.09 (br s, 6H).

### Embodiment 13

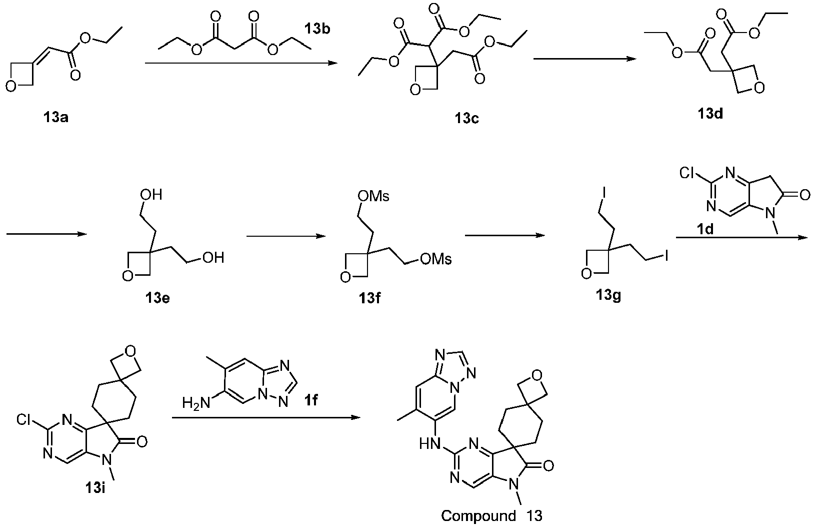

### Step 1

Under the protection of nitrogen at 0 °C, compound **13b** (16.82g, 105.00 mmol, 15.87 mL, 3.5 *eq*) was slowly added dropwise to a tetrahydrofuran (40 mL) solution of sodium hydride (3.60 g, 90.00 mmol, 60 % purity, 3 *eq);* after the addition was completed, the reaction solution was transferred to 20 °C and stirred for 0.5 hours. Then, tetra-*tert*-butyl ammonium chloride (3.34 g, 12.00 mmol, 3.36 mL, 0.4 *eq)* and compound **13a** (4.26 g, 30 mmol, 1 *eq*) were added dropwise to the reaction solution, and the reaction solution was stirred continuously at 20 °C for 18 hours. After the reaction was completed, the reaction solution was quenched with 80 mL of water and extracted with ethyl acetate (50 mL^{∗}3), washed with 50 mL of saturated brine, dried with anhydrous sodium sulfate, filtered, and the filtrate was concentrated under reduced pressure to obtain a crude product, then purified by silica gel column chromatography (ethyl acetate: petroleum ether= 1: 3) to obtain compound **13c**. MS: *m*/*z* 302.9 [M+H]⁺.

### Step 2

Sodium chloride (2.71 g, 46.31 mmol, 2 *eq*) was added to a mixed dimethyl sulfoxide (70 mL) and water (0.7 mL) solution of compound **13c** (7 g, 23.15 mmol, 1 *eq);* and after the addition was completed, the reaction solution was reacted at 160 °C for 5 hours. After the reaction was completed, the reaction solution was diluted with water (50 mL), extracted with ethyl acetate (50 mL), washed with saturated brine (20 mL), dried with anhydrous sodium sulfate, filtered, and the filtrate was concentrated under reduced pressure to obtain a crude product, and purified by silica gel column chromatography (ethyl acetate: petroleum ether= 1: 2) to obtain compound **13d**.

¹H NMR (400 MHz, CDCl₃) δ ppm 4.55 (s, 4H), 4.14 (q, *J*=7.2 Hz, 4H), 2.91 (s, 4H), 1.25 (t, *J*=7.2 Hz, 6H).

### Step 3

Lithium aluminum tetrahydride (455.4 mg, 12.00 mmol, 3 *eq*) was slowly added to an anhydrous tetrahydrofuran (20 mL) solution of compound **13d** (921.0 mg, 4 mmol, 1 *eq)* at - 20 °C; after the addition was completed, the reaction solution was transferred to 20 °C and reacted for 2 hours. After the reaction was completed, water (0.5 mL) was added at 0 °C for extraction, and the mixture was diluted with dichloromethane (20 mL), dried with anhydrous sodium sulfate, filtered, and the filtrate was concentrated under reduced pressure to obtain crude compound **13e**.

¹H NMR (400 MHz, CDCl₃) δ: 4.50 (s, 4H), 3.79 (t, *J*=6.0 Hz, 4H), 2.07 (t, *J*=6.0 Hz 4H), 1.90 (br s, 2H).

### Step 4

Triethylamine (138.4 mg, 1.37 mmol, 190.43 µL, 4 *eq*) and chloromethyl sulfone (117.5 mg, 1.02 mmol, 79 µL, 3 *eq)* were added to a dichloromethane (5 mL) solution of compound **13e** (50 mg, 342.04 µmol, 1 *eq)* at 0 °C; and after the addition was completed, the reaction solution was reacted at 20 °C for 1 hour. After the reaction was completed, the reaction solution was diluted with water (20 mL), extracted with dichloromethane (20 mL), washed with saturated brine (10 mL), dried with anhydrous sodium sulfate, filtered, and the filtrate was concentrated under reduced pressure to obtain crude compound **13f**.

¹H NMR (400 MHz, CDCl₃) δ: 4.49 (s, 4H), 4.35 (t, *J*=6.0 Hz, 4H), 3.03 (s, 6H), 2.26 (t, *J*=6.0 Hz, 4H).

### Step 5

Sodium iodide (470.9 mg, 3.14 mmol, 5 *eq)* was added to an acetone (6 mL) solution of compound **13f** (190 mg, 628.38 µmol, 1 *eq*); after the addition was completed, the reaction solution was reacted at 60 °C for 10 hours. After the reaction was completed, the reaction solution was filtered, and the filtrate was diluted with ethyl acetate (50 mL), washed with water (20 mL), dried with anhydrous sodium sulfate, filtered, and the filtrate was concentrated under reduced pressure and purified by column chromatography (ethyl acetate: petroleum ether= 1: 2) to obtain compound **13g**.

¹H NMR (400 MHz, CDCl₃) δ ppm 4.45 (s, 4H), 3.17(t, *J*=8.0 Hz, 4H), 2.35 (t, *J*=8.0 Hz, 4H).

### Step 6

Cesium carbonate (425.9 mg, 1.31 mmol, 4 *eq)* was added to a *N,N-*dimethylformamide (10 mL) solution of compound **13g** (358.8 mg, 980.42 µmol, 3 *eq*) and compound **Id** (60 mg, 326.81 µmol, 1 *eq);* and after the addition was completed, the reaction solution was reacted at 50 °C for 12 hours. After the reaction was completed, the reaction solution was diluted with water (30 mL), extracted with ethyl acetate (30 mL), dried with anhydrous sodium sulfate, filtered, and the filtrate was concentrated under reduced pressure and purified by silica gel column chromatography (ethyl acetate: petroleum ether= 1: 1) to obtain compound **13i**. MS: *m*/*z* 293.9 [M+H]⁺.

### Step 7

Compound **13i** (32 mg, 108.94 µmol, 1 *eq*), compound **If** (19.4 mg, 130.72 µmol, 1.2 *eq*), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (19.7 mg, 21.79 µmol, 0.2 *eq)* and cesium carbonate (71.0 mg, 217.87 µmol, 2 *eq)* were placed in a reaction flask, and the system was replaced with nitrogen for three times, then 2 mL of dioxane and water (0.2 mL) were added to the mixture and reacted at 100 °C for 1 hour. After the reaction was completed, the mixture was filtered through diatomite, and the filtrate was concentrated under reduced pressure to obtain a crude product, and purified by preparative high performance liquid chromatography (Welch Xtimate C18 100^{∗}40 mm^{∗}3 µm; mobile phase: [water (0.225 % formic acid)-acetonitrile]; acetonitrile %: 13 %-43 %, 8 minutes) to obtain compound **13**. MS: m/z 406.3 [M+H]⁺.

¹H NMR (400 MHz, CDCl₃) δ: 9.72 (s, 1H), 8.26 (s, 1H), 7.94 (s, 1H), 7.59(s, 1H), 6.78 (s, 1H), 4.56-4.58 (m, 4H), 3.24 (s, 3H), 2.52 (s, 3H), 2.26-2.30 (m, 4H), 1.81-1.86(m, 2H), 1.67-1.72(m, 2H).

### Embodiment 14

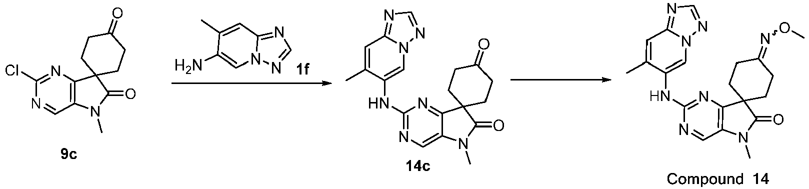

### Step 1

Compound **If** (61.3 mg, 414.01 µmol, 1.1 *eq),* cesium carbonate (183.9 mg, 564.56 µmol, 1.5 *eq*) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (68.2 mg, 75.27 µmol, 0.2 *eq*) were sequentially added to a dioxane (5 mL) solution of compound **9c** (100 mg, 376.37 µmol, 1 *eq);* after the addition was completed, the reaction solution was replaced with nitrogen for three times, then reacted at 100 °C for 3 hours under the protection of nitrogen. After the reaction was completed, the mixture was cooled to 30 °C, diluted with ethyl acetate (20 mL), filtered through diatomite, washed with ethyl acetate, and the obtained filtrate was concentrated under reduced pressure to obtain a crude product, and purified by preparative thin layer chromatography (pure ethyl acetate) to obtain compound **14c**. MS: *m*/*z* 378.0 [M+H]⁺.

¹H NMR (400 MHz, CDCl₃): δ ppm 9.77 (s, 1H), 8.29 (s, 1H), 8.03 (s, 1H), 7.61 (s, 1H), 6.82 (s, 1H), 3.32 (s, 3H), 2.85-2.95 (m, 4H), 2.55 (s, 3H), 2.25-2.40 (m, 2H), 2.15-2.25 (m, 2H).

### Step 2

Methoxyamine hydrochloride (14.9 mg, 178.86 µmol, 13.58 µL, 1.5 *eq*) was added to a pyridine (1 mL) solution of compound **14c** (45 mg, 119.24 µmol, 1 *eq);* after the addition was completed, the reaction solution was reacted at 25 °C for 10 hours. After the reaction was completed, the reaction solution was diluted with water (30 mL), extracted with ethyl acetate (20 mL), washed sequentially with 1 M hydrochloric acid (10 mL) and saturated brine (10 mL), dried with anhydrous sodium sulfate, filtered, and the filtrate was concentrated under reduced pressure to obtain a crude product, and purified by preparative thin layer chromatography (pure ethyl acetate) to obtain compound **14**. MS: *m*/*z* 407.2 [M+H]⁺.

¹H NMR (400 MHz, CDCl₃): δ ppm 9.76 (s, 1H), 8.26 (s, 1H), 7.96 (s, 1H), 7.58 (s, 1H), 6.78 (s, 1H), 3.88 (s, 3H), 3.26 (s, 3H), 3.00-3.10 (m, 1H), 2.85-2.95 (m, 1H), 2.65-2.85 (m, 2H), 2.51 (s, 3H), 1.85-2.15 (m, 4H).

### Embodiment 15

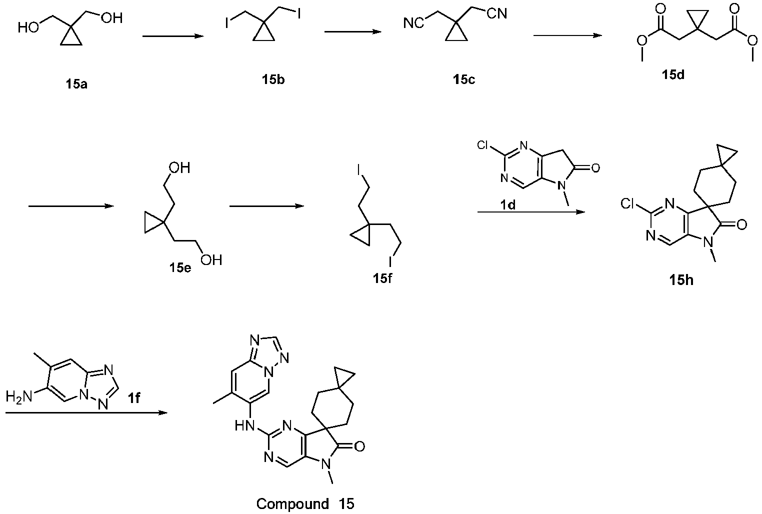

### Step 1

At 0 °C, iodine (19.88 g, 78.33 mmol, 4 *eq*) was added to a dichloromethane (140 mL) solution of imidazole (10.67 g, 156.66 mmol, 8 *eq*) and triphenylphosphine (20.55 g, 78.33 mmol, 4 *eq*)*.* After the addition was completed, the reaction solution was reacted at 0 °C for 1 hour. Then at 0 °C, a dichloromethane (10 mL) solution of compound **15a** (2 g, 19.58 mmol, 1 *eq)* was added. After the addition was completed, the reaction solution was reacted at 30 °C for 2 hours. After the reaction was completed, the reaction solution was diluted with water (100 mL), extracted with dichloromethane (50 mL^{∗}2), dried with anhydrous sodium sulfate, filtered, and the filtrate was concentrated under reduced pressure and purified by column chromatography (ethyl acetate: petroleum ether= 0: 1) to obtain compound **15b**.

¹H NMR (400 MHz, CDCl₃) δ ppm 3.29 (s, 4H), 0.97 (s, 4H).

### Step 2

Potassium cyanide (3.37 g, 51.54 mmol, 3 *eq)* was added to a *N,N*-dimethylformamide (40 mL) solution of compound **15b** (5.55 g, 17.24 mmol, 1 *eq);* and after the addition was completed, the reaction solution was reacted at 80 °C for 16 hours. After the reaction was completed, the reaction solution was cooled to room temperature, diluted with water (300 mL), extracted with ethyl acetate (100 mL^{∗}3), washed sequentially with water (200 mL) and saturated brine (200 mL), dried with anhydrous sodium sulfate, filtered, and the filtrate was concentrated under reduced pressure to obtain compound **15c**.

¹H NMR (400 MHz, CDCl₃) δ ppm 2.57 (s, 4H), 0.81 (m, 4H).

### Step 3

Concentrated sulfuric acid (10.3 g, 105.06 mmol, 5.6 mL) was added to an anhydrous methanol (13 mL) solution of compound **15c** (200 mg, 1.66 mmol, 1 *eq);* after the addition was completed, the reaction solution was reacted at 60 °C for 16 hours. After the reaction was completed, the reaction solution was cooled to room temperature, poured into ice water, extracted with ethyl acetate (200 mL^{∗}2), washed sequentially with water (100 mL) and saturated brine (100 mL), dried with anhydrous sodium sulfate, filtered, and the filtrate was concentrated under reduced pressure to obtain compound **15d**.

¹H NMR (400 MHz, CDCl₃) δ ppm 3.68 (s, 6H), 2.42 (s, 4H), 0.55 (s, 4 H).

### Step 4

Lithium aluminum tetrahydride (1.33 g, 35.02 mmol, 4 *eq*) was added to a tetrahydrofuran (15 mL) solution of compound **15d** (0.3 g, 1.18 mmol, 1 *eq*) at 0 °C; after the addition was completed, the reaction solution was reacted at 25 °C for 16 hours. After the reaction was completed, the reaction solution was cooled to 0 °C, and water (1.33 mL), 20 % sodium hydroxide solution (1.33 mL) and water (4 mL) were sequentially added; after the addition was completed, the mixture was stirred at room temperature for 30 minutes, filtered, and the filtrate was concentrated under reduced pressure to obtain compound **15e**.

¹H NMR (400 MHz, DMSO-*d*₆) δ ppm 4.31 (br t, *J*=4.69 Hz, 2H), 3.42-3.49 (m, 4H), 1.34-1.40 (m, 4H), 0.21 (s, 4H).

### Step 5

At 0 °C, iodine (8.50 g, 33.49 mmol, 4 *eq*) was added to a dichloromethane (70 mL) solution of imidazole (4.56 g, 66.98 mmol, 8 *eq*) and triphenylphosphine (8.78 g, 33.49 mmol, *4 eq).* After the addition was completed, the reaction solution was reacted at 0 °C for 1 hour. Then a dichloromethane (4 mL) solution of compound **15e** (1.09 g, 8.37 mmol, 1 *eq)* was added, after the addition was completed, the reaction solution was reacted at 30 °C for 2 hours. After the reaction was completed, the reaction solution was diluted with water (200 mL), extracted with dichloromethane (100 mL), dried with anhydrous sodium sulfate, filtered, and the filtrate was concentrated under reduced pressure and purified by column chromatography (ethyl acetate: petroleum ether= 0: 1) to obtain compound **15f**.

¹H NMR (400 MHz, DMSO-*d*₆) δ ppm 3.22-3.28 (t, *J*=8 Hz, 4H) 1.75-1.83 (t, *J*=8 Hz, 4H) 0.38 (s, 4H).

### Step 6

Compound **Id** (1.39 g, 3.97 mmol, 2 *eq)* and cesium carbonate (1.4 g, 5.96 mmol, 3 *eq*) were sequentially added to a *N,N*-dimethylformamide (15 mL) solution of compound **15f** (0.364 g, 1.99 mmol, 1 *eq*); and after the addition was completed, the reaction solution was reacted at 100 °C for 16 hours. After the reaction was completed, the reaction solution was cooled to room temperature, diluted with water, extracted with ethyl acetate (50 mL^{∗}2), washed sequentially with water (200 mL) and saturated brine (200 mL), dried with anhydrous sodium sulfate, filtered, and the filtrate was concentrated under reduced pressure and purified by column chromatography (ethyl acetate: petroleum ether= 0:1 to 1:1) to obtain compound **15h**. MS: *m*/*z.* 277.9 [M+H]⁺;

¹H NMR (400 MHz, CDCl₃) δ ppm 8.04 (s, 1H), 3.26 (s, 3H), 1.88-1.97 (m, 4H), 1.24-1.28 (m, 4H), 0.29-0.45 (m, 4H).

### Step 7

Compound **If** (109.8 mg, 741.32 µmol, 1 *eq*), cesium carbonate (362.3 mg, 1.11 mmol, 1.5 *eq)* and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (134.4 mg, 148.26 µmol, 0.2 *eq*) were sequentially added to a dioxane (2 mL) solution of compound **15h** (205.9 mg, 741.32 mmol, 1 *eq);* after the addition was completed, the reaction solution was replaced with nitrogen for three times, then reacted at 100 °C for 3 hours under the protection of nitrogen. After the reaction was completed, the mixture was cooled to room temperature, filtered through diatomite, washed with ethyl acetate (50 mL), and the filtrate was concentrated under reduced pressure to obtain a crude product, and purified by preparative high performance liquid chromatography (Welch Xtimate C18 100^{∗}40 mm^{∗}3 µm; mobile phase: [water (0.225 % formic acid)-acetonitrile]; B (acetonitrile) %: 40 %-50 %, 8 minutes) to obtain compound **15**. MS: *m*/*z* 390.3 [M+H]⁺;

¹H NMR (400 MHz, CDCl₃) δ ppm 9.80 (s, 1H), 8.26 (s, 1H), 7.93 (s, 1H), 7.58 (s, 1H), 6.82 (s, 1H), 3.25 (s, 3H), 2.54 (s, 3H), 1.89-2.10 (m, 4H), 1.72-1.81 (m, 2H), 1.44-1.52 (m, 2H), 0.32-0.49 (m, 4H).

### Embodiment 16

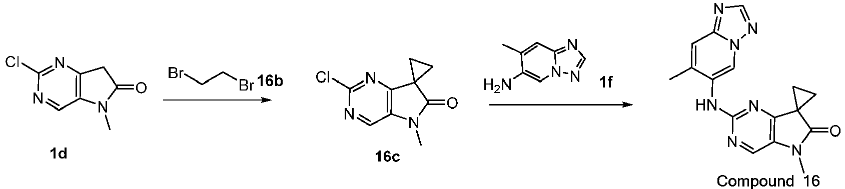

### Step 1

At 30 °C, compound **16b** (409 mg, 2.18 mmol, 2 *eq*) and potassium carbonate (376.39 mg, 2.72 mmol, 2.5 *eq*) were sequentially added to a *N,N*-dimethylformamide (3 mL) solution of compound **Id** (200 mg, 1.09 mmol, 1 *eq*); and after the addition was completed, the reaction solution was reacted at 80 °C for 2 hours. After the reaction was completed, the reaction solution was cooled to room temperature, diluted with water (20 mL), and extracted with ethyl acetate (20 mL^{∗}3). The organic phases were combined, washed with water (20 mL^{∗}3) and saturated brine (20 mL^{∗}2), dried with anhydrous sodium sulfate, filtered, and the filtrate was concentrated under reduced pressure and purified by preparative thin layer chromatography (ethyl acetate: petroleum ether= 1:1) to obtain compound **16c**. MS: m/z 209.9 [M+H]⁺.

### Step 2

Compound **If** (14.14 mg, 95.41 µmol, 1 *eq*), cesium carbonate (46.63 mg, 143.11 µmol, 1.5 *eq*) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (17.03 mg, 19.08 µmol, 0.2 *eq*) were added to a dioxane (1 mL) solution of compound **16c** (20 mg, 95.41 µmol, 1 *eq*), and the reaction solution was replaced with nitrogen for three times, then reacted at 100 °C for 3 hours under the protection of nitrogen. After the reaction was completed, the reaction solution was cooled to room temperature, diluted with ethyl acetate (20 mL), filtered through diatomite, washed with ethyl acetate, and the obtained filtrate was concentrated under reduced pressure and purified by preparative thin layer chromatography (ethyl acetate: petroleum ether=1: 0) to obtain compound **16**. MS: m/z 322.2 [M+H]⁺;

¹H NMR (400 MHz, CDCl₃): δ ppm 9.70 (s, 1H), 8.19 (s, 1H), 7.84 (s, 1H), 7.50 (s, 1H), 6.65 (s, 1H), 3.27 (s, 3H), 2.44 (s, 3H), 1.80-1.90 (m, 4H).

### Embodiment 17

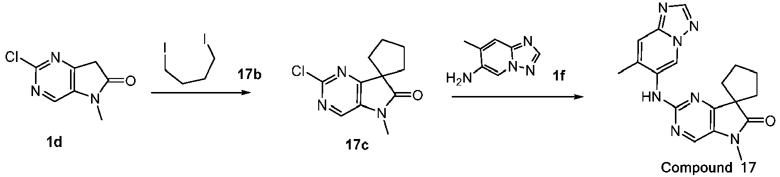

### Step 1

Compound **17b** (337.61 mg, 1.09 mmol, 2 *eq*) and cesium carbonate (532.40 mg, 1.63 mmol, 3 *eq)* were sequentially added to a *N,N*-dimethylformamide (2 mL) solution of compound **Id** (100 mg, 544.68 µmol, 1 *eq),* and the reaction solution was reacted at 80 °C for 16 hours. After the reaction was completed, the reaction solution was cooled to room temperature, diluted with water (20 mL), and extracted with 40 mL of ethyl acetate (20 mL^{∗}2). The organic phases were combined, washed with water (20 mL), dried with anhydrous sodium sulfate, filtered, and the filtrate was concentrated under reduced pressure to obtain a crude product and purified by preparative thin layer chromatography (ethyl acetate: petroleum ether = 1: 2) to obtain compound **17c**. MS: *m*/*z* 237.8 [M+H]⁺;

¹H NMR (400 MHz, CDCl₃) δ ppm 8.00 (s, 1H), 3.25 (s, 3H), 2.00-2.16 (m, 8H).

### Step 2

A dioxane (3 mL) solution of compound **17c** (32 mg, 134.63 µmol, **1** *eq*), compound **If** (19.95 mg, 134.63 µmol, 1 *eq*), cesium carbonate (65.80 mg, 201.95 µmol, 1.5 *eq*) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (24.41 mg, 26.93 µmol, 0.2 *eq)* was replaced with nitrogen for three times under the protection of nitrogen, and then the reaction solution was reacted at 100 °C for 3 hours under the protection of nitrogen. After the reaction was completed, the reaction solution was cooled to 20 °C, filtered through diatomite, and the filter cake was washed with ethyl acetate (10 mL), and the obtained filtrate was concentrated under reduced pressure to obtain a crude product, and purified by preparative thin layer chromatography (dichloromethane: methanol= 20: 1) to obtain compound **17**. MS: m/z 350.2 [M+H]⁺;

¹H NMR (400 MHz, CDCl₃) δ ppm 9.80 (s, 1H), 8.26 (s, 1H), 7.89 (s, 1H), 7.57 (s, 1H), 6.77 (s, 1H), 3.25 (s, 3H), 2.52 (s, 3H), 1.99-2.19 (m, 8H).

### Embodiments 18, 19

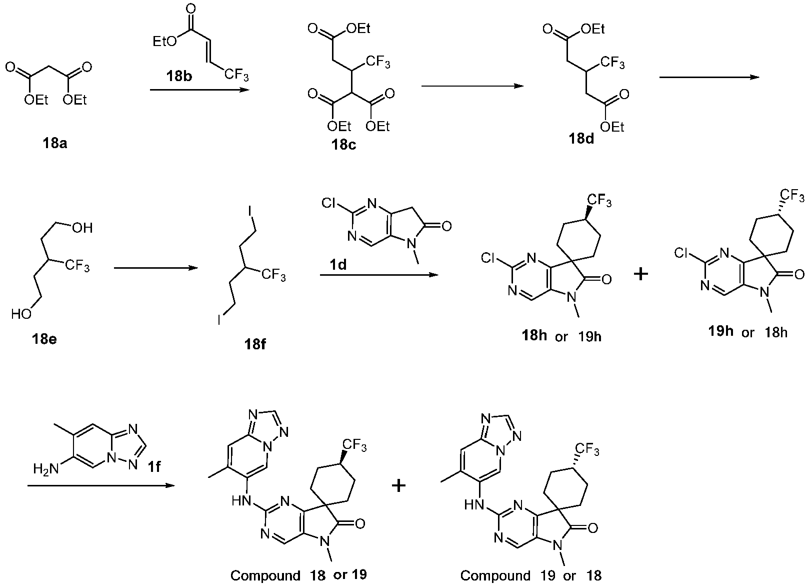

### Step 1

Sodium hydride (1.2 g, 30 mmol, 1 *eq*, 60 % purity) was added to a tetrahydrofuran (15 mL) solution of compound **18a** (4.81 g, 30 mmol, 1 *eq*) at 0 °C; and after the addition was completed, the reaction mixture was reacted for 0.5 hours at 0 °C; then compound 18b (5.04 g, 30 mmol, 1 *eq)* was added to the reaction solution, and after the addition was completed, the reaction solution was reacted for 2 hours at 20 °C. After the reaction was completed, the reaction solution was quenched with water (20 mL) and extracted with 90 mL of ethyl acetate (30 mL^{∗}3), washed with saturated brine (20 mL), dried with anhydrous sodium sulfate, and concentrated under reduced pressure and purified by rapid column chromatography (ethyl acetate: petroleum ether= 0: 1 to 1:4) to obtain compound 18c.

¹H NMR (400 MHz, CDCl₃) δ ppm 4.12-4.29 (m, 6H), 3.77 (d, *J*=5.63 Hz, 1H), 3.60-3.72 (m, 1H), 2.80-2.92 (m, 1H), 2.65-2.78 (m, 1H), 1.20-1.35 (m, 9H).

### Step 2

Lithium chloride (635.91 mg, 15 mmol, 1 *eq*) was added to a mixed dimethyl sulfoxide (13 mL) and water (0.8 mL) solution of compound **18c** (4.92 g, 15 mmol, 1 *eq*); after the addition was completed, the reaction solution was reacted at 160 °C for 3 hours. After the reaction was completed, the reaction solution was diluted with water (50 mL), and extracted with 90 mL of ethyl acetate (30 mL^{∗}3), washed with saturated brine (20 mL), dried with anhydrous sodium sulfate, and concentrated under reduced pressure and purified by rapid column chromatography (ethyl acetate: petroleum ether= 0: 1 to 1: 4) to obtain compound **18d.**

¹H NMR (400 MHz, CDCl₃) δ ppm 4.16 (q, *J*=7.13 Hz, 4H), 3.21-3.37 (m, 1H), 2.61-2.74 (m, 2H), 2.45-2.55 (m, 2H), 1.26 (t, *J*=7.13 Hz, 6H).

### Step 3

Lithium aluminum tetrahydride (313.12 mg, 8.25 mmol, 1.5 *eq*) was added to a tetrahydrofuran (20 mL) solution of compound **18d** (1.41 g, 5.5 mmol, 1 *eq)* at 0 °C; after the addition was completed, the reaction solution was reacted at 20 °C for 4 hours. After the reaction was completed, the reaction solution was sequentially added with water (0.35 mL), 20 % sodium hydroxide (0.35 mL), and water (1.05 mL) at 0 °C; after the addition was completed, the mixture was transferred to 20 °C and stirred for 0.5 hours, and then anhydrous sodium sulfate was added and stirred continuously for 0.5 hours. The mixture was filtered, and the filtrate was concentrated under reduced pressure to obtain crude compound **18e.**

¹H NMR (400 MHz, CDCl₃) δ ppm 3.70-3.90 (m, 4H), 2.43-2.60 (m, 1H), 2.22 (br s, 2H), 1.88-2.00 (m, 2H), 1.62-1.73 (m, 2H).

### Step 4

At 0 °C, imidazole (0.794 g, 11.66 mmol, 2.2 *eq*)*,* triphenylphosphine (2.78 g, 10.6 mmol, 2 *eq*) and iodine (2.69g, 10.6mmol, 2 *eq)* were sequentially added to a tetrahydrofuran (20 mL) solution of compound **18e** (0.912 g, 5.3 mmol, 1 *eq*)*,* and the reaction solution was first reacted at 0 °C for 1 hour, and then reacted at 20 °C for 4 hours. After the reaction was completed, the reaction solution was quenched with saturated sodium thiosulfate solution (30 mL) and extracted with 200 mL of ethyl acetate (100 mL^{∗}2), washed with saturated brine (30 mL), dried with anhydrous sodium sulfate, and concentrated under reduced pressure and purified by rapid column chromatography (ethyl acetate: petroleum ether= 0: 1 to 1: 4) to obtain compound **18f.**

¹H NMR (400 MHz, CDCl₃) δ ppm 3.16-3.34 (m, 4H), 2.34-2.47 (m, 1H), 2.22 (dq, *J*=14.56, 7.11 Hz, 2H), 1.95 (dq, *J*=14.37, 7.13 Hz, 2H).

### Step 5

Cesium carbonate (1.08 g, 3.3 mmol, 3 *eq*) and compound **18f** (0.862 g, 2.2 mmol, 2 *eq*) were sequentially added to a *N,N*-dimethylformamide (20 mL) solution of compound **Id** (0.202 g, 1.1 mmol, 1 *eq*)*,* and the reaction solution was reacted at 80 °C for 6 hours. After the reaction was completed, the reaction solution was diluted with water (25 mL) and extracted with 150 mL of ethyl acetate (50 mL^{∗}3), washed with saturated brine (30 mL), dried with anhydrous sodium sulfate, and concentrated under reduced pressure and purified by rapid column chromatography (ethyl acetate: petroleum ether= 0: 1 to 1: 1) to obtain compound **18h** and **19h.**

**18h:** (Rf value was 0.6, ethyl acetate: petroleum ether=1: 1), MS: m/z 319.9 [M+H]⁺;

¹H NMR (400 MHz, CDCl₃) δ ppm 8.04 (s, 1H), 3.24 (s, 3H), 2.17-2.28 (m, 2H), 1.91-2.10 (m, 5H), 1.76-1.89 (m, 2H).

**19h:** (Rf value was 0.4, ethyl acetate: petroleum ether=1: 1), MS: m/z 319.9 [M+H]⁺;

¹H NMR (400 MHz, CDCl₃) δ ppm 8.09 (s, 1H), 3.27 (s, 3H), 2.18-2.32 (m, 3H), 1.92-2.01 (m, 4H), 1.70-1.80 (m, 2H).

### Step 6

Compound **18h** (25.58 mg, 80 µmol, 1 *eq*)*,* compound **If** (10.67 mg, 72 µmol, 0.9 *eq*)*,* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (14.5 mg, 16 µmol, 0.2 *eq)* and cesium carbonate (39.10 mg, 120 µmol, 2 *eq)* were placed in a reaction flask, and the system was replaced with nitrogen for three times, then anhydrous dioxane (2 mL) was added to the mixture and reacted at 100 °C for 3 hours. After the reaction was completed, the reaction solution was concentrated under reduced pressure and purified by column chromatography (methanol: dichloromethane = 0: 1 to 1: 9) and preparative thin layer chromatography (methanol: dichloromethane = 1: 15) to obtain compound **18.** MS: *m*/*z* 432.2 [M+H]⁺;

¹H NMR (400 MHz, CDCl₃) δ ppm 9.76 (s, 1H), 8.27 (s, 1H), 7.92 (s, 1H), 7.59 (s, 1H), 6.79 (s, 1H), 3.24 (s, 3H), 2.52 (s, 3H), 2.22-2.35 (m, 3H), 1.85-1.99 (m, 6H).

Compound **19h** (25.58 mg, 80 µmol, 1 *eq*)*,* compound **18i** (10.67 mg, 72 µmol, 0.9 *eq*)*,* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (14.5 mg, 16 µmol, 0.2 *eq*) and cesium carbonate (39.10 mg, 120 µmol, 1.5 *eq)* were placed in a reaction flask, and the system was replaced with nitrogen for three times, then anhydrous dioxane (2 mL) was added to the mixture and reacted at 100 °C for 3 hours. After the reaction was completed, the reaction solution was concentrated under reduced pressure and purified by column chromatography (methanol: dichloromethane = 0: 1 to 1: 9) and preparative thin layer chromatography (methanol: dichloromethane = 1: 15) to obtain compound **19.** MS: *m*/*z* 432.3 [M+H]⁺;

¹H NMR (400 MHz, CDCl₃) δ ppm 9.61 (s, 1H), 8.27 (s, 1H), 7.96 (s, 1H), 7.59 (s, 1H), 6.74 (s, 1H), 3.26 (s, 3H), 2.50 (s, 3H), 2.19-2.31 (m, 3H), 1.91-2.04 (m, 4H), 1.71-1.82 (m, 2H).

### Embodiment 20

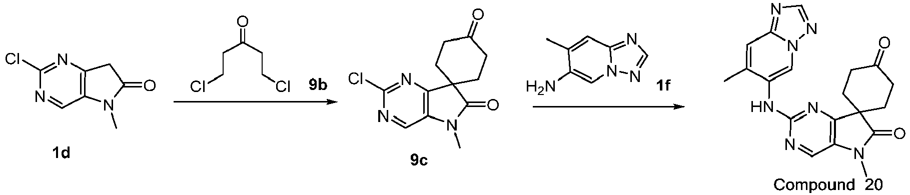

### Step 1

Cesium carbonate (1.42 g, 4.36 mmol, 4 *eq*)*,* sodium iodide (163.29 mg, 1.09 mmol, 1 *eq*) and compound **9b** (506.62 mg, 3.27 mmol, 3 *eq*) were sequentially added to a *N,N-*dimethylformamide (5 mL) solution of compound **Id** (200 mg, 1.09 mmol, 1 *eq*); and after the addition was completed, the reaction solution was reacted at 60 °C for 14 hours. After the reaction was completed, the reaction solution was cooled to room temperature, diluted with water, extracted with 100 mL of ethyl acetate (50 mL^{∗}2); the organic phases were combined, washed sequentially with water (100 mL) and saturated brine (100 mL), dried with anhydrous sodium sulfate, filtered, and the filtrate was concentrated under reduced pressure to obtain crude compound **9c.** MS: *m*/*z*. 266.1 [M+H]⁺;

¹H NMR (400 MHz, CDCl₃) δ ppm 8.14 (s, 1H), 3.31 (s, 3H), 2.87-2.96 (m, 2H), 2.73-2.82 (m, 2H), 2.23-2.32 (m, 2H), 2.12-2.21 (m, 2H).

### Step 2

Compound **If** (33.91 mg, 228.83 µmol, 0.8 *eq*)*,* cesium carbonate (139.8 mg, 429.06 µmol, 1.5 *eq*) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (51.86 mg, 57.21 µmol, 0.2 *eq*) were added to a dioxane (2 mL) solution of compound **9c** (76 mg, 286.04 µmol, 1 *eq),* and the reaction solution was replaced with nitrogen for three times, then reacted at 100 °C for 3 hours under the protection of nitrogen. After the reaction was completed, the reaction solution was cooled to room temperature, filtered through diatomite, washed with ethyl acetate (50 mL), and the obtained filtrate was concentrated under reduced pressure and purified by preparative thin layer chromatography (dichloromethane: methanol= 20: 1) to obtain compound **20.** MS: *m*/*z* 378.1 [M+H]⁺;

¹H NMR (400 MHz, CDCl₃) δ ppm 9.75 (s, 1H), 8.27 (s, 1H), 8.01 (s, 1H), 7.59 (s, 1H), 6.79 (s, 1H), 3.30 (s, 3H), 2.85-2.93 (m, 4H), 2.55 (s, 3H), 2.25-2.33(m, 2H), 2.14-2.22 (m, 2H).

### Embodiments 21, 22

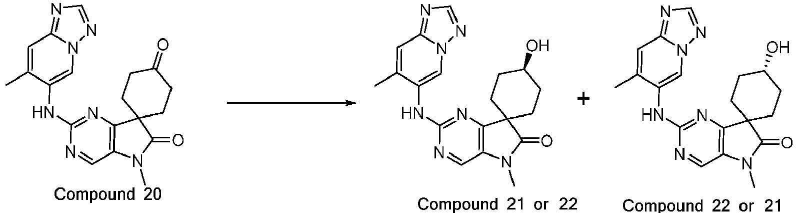

### Step 1

At 0 °C, sodium borohydride (3.01 mg, 79.49 µmol, 1 *eq)* was added to a methanol solution (5 mL) of compound **20** (30 mg, 79.49 µmol, 1 *eq);* after the addition was completed, the reaction solution was reacted at 20 °C for 1 hour. After the reaction was completed, the reaction solution was added dropwise with saturated ammonium chloride solution (2 mL) to quench the reaction, extracted with 30 mL of ethyl acetate (10 mL^{∗}3). The organic phases were combined, washed with water (50 mL), dried with anhydrous sodium sulfate, filtered, and the filtrate was concentrated under reduced pressure and purified by supercritical fluid chromatography (DAICEL CHIRALCEL OD-H (250 mm ^{∗} 30 mm ^{∗} 5 µm); mobile phase: [0.1 % ammonia water/ethanol]; (0.1 % ammonia water/ethanol) %: 45 %-45 %, min) to obtain compound **21** (retention time was 5.288 minutes) and compound **22** (retention time was 5.826 minutes).

### Compound 21: MS: m/z 380.3 [M+H]⁺;

¹H NMR (400 MHz, CDCl₃) δ ppm 10.13 (s, 1H), 8.27 (s, 1H), 8.00 (s, 1H), 7.59 (s, 1H), 6.92 (s, 1H), 3.80-4.04 (m, 1H), 3.27 (s, 3H), 2.85 (s, 1H), 2.55 (s, 3H), 2.17-2.27 (m, 4H), 2.00-2.10 (m, 2H), 1.77-1.86 (m, 2H).

### Compound 22: MS: m/z 380.3 [M+H]⁺;

¹H NMR (400 MHz, CDCl₃) δ ppm 9.77 (s, 1H), 8.28 (s, 1H), 7.94 (s, 1H), 7.59 (s, 1H), 6.78 (s, 1H), 4.02-4.08 (m, 1H), 3.50 (s, 1H), 3.26 (s, 3H), 2.53 (s, 3H) 2.16-2.25 (m, 2H), 2.04-2.12 (m, 4H), 1.75-1.83 (m, 2H).

### Embodiment 23

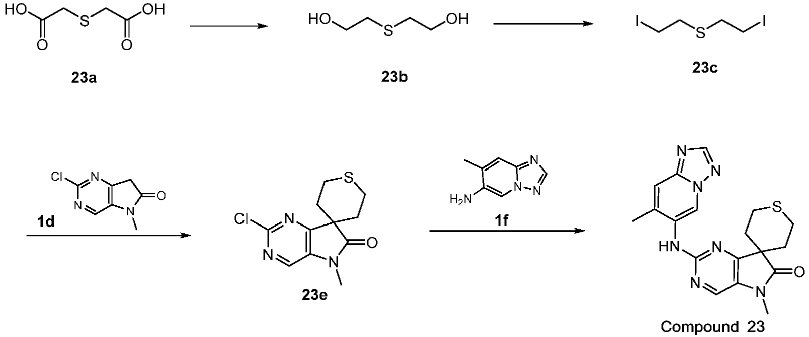

### Step 1

Lithium aluminum tetrahydride (2.53 g, 66.60 mmol, 4 *eq*) was added to a tetrahydrofuran (100 mL) solution of compound **23a** (2.5 g, 16.25 mmol, 1 *eq)* at 0 °C; after the addition was completed, the reaction solution was reacted at 20 °C for 16 hours. After the reaction was completed, tetrahydrofuran (100 mL) was added to dilute and the reaction solution was cooled to 0 °C, and water (2.5 mL), 20 % sodium hydroxide solution (2.5 mL) and water (7.5 mL) were sequentially added to the reaction solution, and then the mixture was stirred at room temperature for 30 minutes. The reaction solution was filtered, and the filtrate was concentrated under reduced pressure to obtain crude compound **23b.**

¹H NMR (400 MHz, CDCl₃): δ ppm 3.70-3.80 (m, 4H), 2.72-2.80 (m, 4H), 2.47 (br s, 2H).

### Step 2

At 0 °C, iodine (5.82 g, 22.92 mmol, 4 *eq*) was added to a dichloromethane (300 mL) solution of imidazole (3.12 g, 45.83 mmol, 8 *eq*) and triphenylphosphine (6.01 g, 22.92 mmol, *4 eq);* after the addition was completed, the reaction solution was reacted at 0 °C for 1 hour. Then compound **23b** (0.7 g, 5.73 mmol, 1 *eq)* was added to the reaction solution at 0 °C; after the addition was completed, and the reaction solution was reacted at 20 °C for 14 hours. After the reaction was completed, the reaction solution was diluted with water (400 mL), extracted with dichloromethane (100 mL^{∗}2); the organic phases were combined, dried with anhydrous sodium sulfate, filtered, and the filtrate was concentrated under reduced pressure and purified by column chromatography (ethyl acetate: petroleum ether= 0: 1) to obtain compound **23c.**

¹H NMR (400 MHz, CDCl₃): δ ppm 3.26 (t, *J*=8.0 Hz, 4H), 2.99 (t, *J*=8.0 Hz, 4H).

### Step 3

Compound **23c** (372.54 mg, 1.09 mmol, 2 *eq*) and cesium carbonate (532.40 mg, 1.63 mmol, *3eq*) were sequentially added to a *N,N*-dimethylformamide (2 mL) solution of compound **Id** (100 mg, 544.68 µmol, 1 *eq*)*,* and after the addition was completed, the reaction solution was reacted at 60 °C for 16 hours. After the reaction was completed, the reaction solution was cooled to room temperature, diluted with water (10 mL), and extracted with ethyl acetate (20 mL^{∗}3). The organic phases were combined, washed with water (20 mL^{∗}3) and saturated brine (20 mL^{∗}2), dried with anhydrous sodium sulfate, filtered, and the filtrate was concentrated under reduced pressure and purified by preparative thin layer chromatography (ethyl acetate: petroleum ether= 1: 1) to obtain compound **23e.** MS: *m*/*z* 269.9 [M+H]⁺.

### Step 4

Compound **If** (13.73 mg, 92.68 µmol, 1 *eq*)*,* cesium carbonate (45.3 mg, 139.02 µmol, 1.5 *eq*) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (16.8 mg, 18.54 µmol, 0.2 *eq)* were sequentially added to a dioxane (2 mL) solution of compound **23e** (25 mg, 92.68 µmol, 1 *eq),* and the system was replaced with nitrogen for three times, then the reaction solution was reacted at 100 °C for 3 hours under the protection of nitrogen. After the reaction was completed, the reaction solution was cooled to room temperature, diluted with ethyl acetate (20 mL), filtered through diatomite; the filter cake was washed with ethyl acetate, and the obtained filtrate was concentrated under reduced pressure and purified by preparative thin layer chromatography (ethyl acetate: petroleum ether=1: 0) to obtain compound **23.** MS: *m*/*z* 382.1 [M+H]⁺;

¹H NMR (400 MHz, CDCl₃): δ ppm 9.78 (s, 1H), 8.27 (s, 1H), 7.95 (s, 1H), 7.59 (s, 1H), 6.84 (s, 1H), 3.25 (s, 3H), 3.10-3.25 (m, 2H), 2.90-3.00 (m, 2H), 2.54 (s, 3H), 2.10-2.25 (m, 2H), 2.00-2.10 (m, 2H).

### Embodiment 24

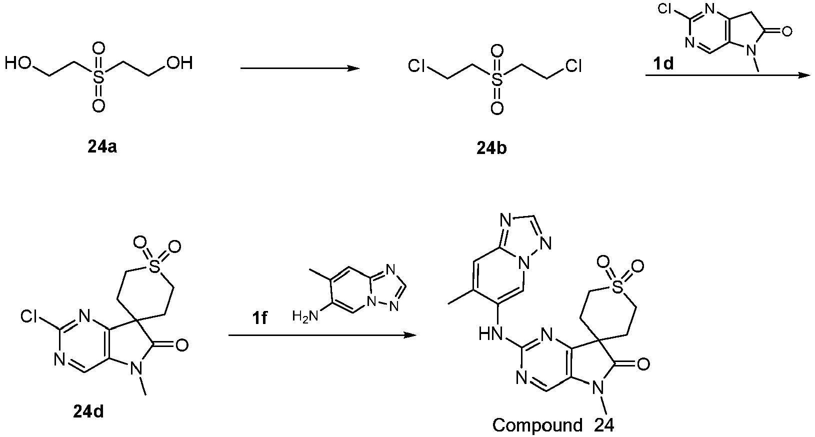

### Step 1

Pyridine (1.03 g, 12.97 mmol, 2 *eq*) and sulfoxide chloride (4.63 g, 36.91 mmol, 6 *eq*) were added to a toluene (30 mL) solution of compound **24a** (1 g, 6.49 mmol, 1 *eq*); and after the addition was completed, the reaction solution was reacted for 3 hours at 110 °C. After the reaction was completed, the reaction solution was cooled to room temperature, added with water (40 mL), extracted with ethyl acetate (20 mL^{∗}3); the organic phases were combined, washed with saturated brine (30 mL^{∗}2), dried with anhydrous sodium sulfate, filtered, and the filtrate was concentrated under reduced pressure to obtain crude compound **24b.**

¹H NMR (400 MHz, CDCl₃): δ ppm 3.93 (t, *J*=6.8 Hz, 4H), 3.54 (t, *J*=6.8 Hz, 4H).

### Step 2

Compound **24b** (416.3 mg, 2.18 mmol, 2 *eq*)*,* sodium iodide (163.3 mg, 1.09 mmol, 2 *eq*) and cesium carbonate (709.8 mg, 2.18 mmol, *2eq)* were sequentially added to a *N,N-*dimethylformamide (2 mL) solution of compound **Id** (200 mg, 1.09 mmol, 1 *eq*) at 25 °C; and after the addition was completed, the reaction solution was reacted at 80 °C for 16 hours. After the reaction was completed, the reaction solution was cooled to room temperature, diluted with water (10 mL), and extracted with ethyl acetate (10 mL^{∗}3). The organic phases were combined, washed with water (10 mL^{∗}3) and saturated brine (20 mL^{∗} 2), dried with anhydrous sodium sulfate, filtered, and the filtrate was concentrated under reduced pressure and purified by preparative thin layer chromatography (methanol: dichloromethane = 1: 10) to obtain compound **24d.**

¹H NMR (400 MHz, CDCl₃): δ ppm 8.15 (s, 1H), 3.50-3.60 (m, 2H), 3.25-3.45 (m, 2H), 3.29 (s, 3H), 2.50-2.60 (m, 2H), 2.30-2.40 (m, 2H).

### Step 3

Compound **If** (34.37 mg, 231.98 µmol, 1.1 *eq*)*,* cesium carbonate (113.38 mg, 347.97 µmol, 1.5 *eq)* and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (42.06 mg, 46.4 µmol, 0.2 *eq*) were added to a dioxane (2 mL) solution of compound **24d** (70 mg, 231.98 µmol, 1 *eq*)*,* and the system was replaced with nitrogen for three times, then the reaction solution was reacted at 100 °C for 3 hours under the protection of nitrogen. After the reaction was completed, the reaction solution was cooled to room temperature, diluted with ethyl acetate (20 mL), filtered through diatomite; the filter cake was washed with ethyl acetate, and the obtained filtrate was concentrated under reduced pressure and purified by preparative thin layer chromatography (methanol: dichloromethane = 1:15 for three times) to obtain compound **24.** MS: *m*/*z* 414.2 [M+H]⁺;

¹H NMR (400 MHz, CDCl₃): δ ppm 9.72 (s, 1H), 8.28 (s, 1H), 8.02 (s, 1H), 7.60 (s, 1H), 6.86 (s, 1H), 3.45-3.60 (m, 4H), 3.28 (s, 3H), 2.54 (s, 3H), 2.40-2.50 (m, 4H).

### Embodiments 25, 26

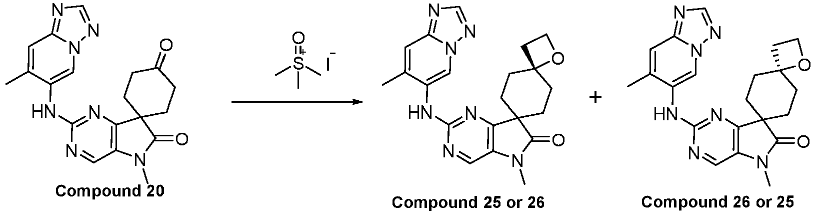

### Step 1

Potassium tert-butoxide (74.33 mg, 662.43 µmol, 2.5 *eq*) and trimethyl sulfoxide iodide (145.78 mg, 662.43 µmol, 2.5 *eq*) were added to a *tert*-butanol (10 mL) solution of compound **20** (100 mg, 264.97 mol, 1 *eq);* and after the addition was completed, the reaction solution was reacted at 60 °C for 4 hours. After the reaction was completed, the reaction solution was diluted with ethyl acetate (30 mL), filtered, and the filtrate was concentrated under reduced pressure and purified by preparative thin layer chromatography (dichloromethane: methanol= 15: 1) to obtain a crude compound. The crude compound was purified by supercritical fluid chromatography (column: DAICEL CHIRALPAK IG (250 mm^{∗}30 mm,10 µm); mobile phase: [0.1 % ammonia water in ethanol]; 0.1 % ammonia water/ethanol %: 50 %-50 %) to obtain compound **25** (retention time was 4.225 minutes) and **26** (retention time was 4.619 minutes).

### Compound 25: MS: m/z 406.2 [M+H]⁺;

¹H NMR (400 MHz, CDCl₃) δ ppm 9.97 (s, 1H), 8.27 (s, 1H), 8.01 (s, 1H), 7.71 (s, 1H), 7.59 (s, 1H), 4.31 (br d, *J*=9.88 Hz, 1H), 3.89-3.97 (m, 1H), 3.76-3.84 (m, 1H), 3.30 (s, 3H), 2.72-2.83 (m, 1H), 2.54 (s, 3H), 2.44-2.50 (m, 1H), 2.28-2.38 (m, 2H), 2.16-2.27 (m, 2H), 2.07-2.15 (m, 1H), 1.97-2.02 (m, 1H), 1.77-1.85 (m, 1H).

### Compound 26: MS: m/z 406.2 [M+H]⁺;

¹H NMR (400 MHz, CDCl₃) δ ppm 9.97 (s, 1H), 8.27 (s, 1H), 8.01 (s, 1H), 7.71 (s, 1H), 7.50-7.64 (m, 1H), 4.31 (br d, *J*=9.76 Hz, 1H), 3.88-3.97 (m, 1H), 3.74-3.83 (m, 1H), 3.30 (s, 3H), 2.72-2.83 (m, 1H), 2.54 (s, 3H), 2.44-2.51 (m, 1H), 2.28-2.39 (m, 2H), 2.16-2.28 (m, 2H), 2.07-2.15 (m, 1H), 1.97-2.04 (m, 1H), 1.74-1.84 (m, 1H).

### Embodiments 27, 28

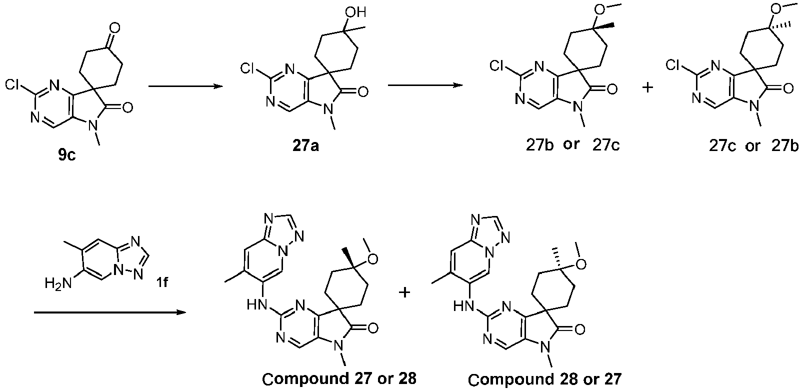

### Step 1

At -60 °C, methylmagnesium bromide (6.78 mmol, 3 mol/L, 2.26 mL, 3 *eq*) was added dropwise to a tetrahydrofuran (60 mL) solution of compound **9c** (600 mg, 2.26 mmol, 1 *eq*); and after the addition was completed, the reaction solution was reacted at -60 °C for 1 hour. After the reaction was completed, the reaction solution was quenched with saturated ammonium chloride (5 mL) solution, concentrated under reduced pressure, diluted with water (15 mL), extracted with ethyl acetate (30 mL^{∗}3), dried with anhydrous sodium sulfate, filtered, and the filtrate was concentrated under reduced pressure and purified by column chromatography (ethyl acetate: petroleum ether = 0: 1 to 1: 1) to obtain compound **27a,** MS: *m*/*z* 281.9 [M+H]⁺;

### Step 2

Sodium hydride (172.93 mg, 4.32 mmol, 60 % content, 3 *eq*) was added to an anhydrous tetrahydrofuran (20 mL) solution of compound **27a** (406 mg, 1.44 mmol, 1 *eq*) in batches, and the mixture was placed in a reaction flask, and then the system was replaced with nitrogen for three times; then methyl iodide (613.63 mg, 4.32 mmol, 3 *eq*) was added to the reaction flask, and the reaction solution was reacted at 80 °C for 4 hours. After the reaction was completed, the reaction solution was cooled to room temperature, quenched with water (5 mL), concentrated under reduced pressure, diluted with water (20 mL), extracted with ethyl acetate (50 mL^{∗}3), dried with anhydrous sodium sulfate, filtered, and the filtrate was concentrated under reduced pressure and purified by preparative thin layer chromatography (ethyl acetate: petroleum ether=1: 1) to obtain compound **27b** (Rf = 0.50, ethyl acetate: petroleum ether=1: 1, MS: m/z 295.9 [M+H]⁺) and **27c** (Rf=0.45, ethyl acetate:petroleum ether=1: 1, MS: *m*/*z* 295.9 [M+H]⁺).

### Step 3

Compound **27b** (30 mg, 101.43 µmol, 1 *eq*)*,* compound **1f** (13.53 mg, 91,29 µmol, 0.9 *eq*)*,* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (18.39 mg, 20.29 µmol, 0.2 *eq*) and cesium carbonate (49.57 mg, 152.15 µmol, 1.5 *eq*) were placed in a reaction flask, and the system was replaced with nitrogen for three times, then anhydrous dioxane (2 mL) was added to the mixture and reacted at 100 °C for 1.5 hours. After the reaction was completed, the reaction solution was concentrated under reduced pressure and purified by preparative thin layer chromatography (eluted successively by dichloromethane: methanol = 20: 1 and ethyl acetate: petroleum ether = 1: 0) to obtain compound **27,** MS: *m*/*z* 408.2 [M+H]⁺.

¹H NMR (400 MHz, CDCl₃) δ ppm 9.83 (s, 1H), 8.26 (s, 1H), 7.89 (s, 1H), 7.57 (s, 1H), 6.88 (s, 1H), 3.29 (s, 3H), 3.22 (s, 3H), 2.53 (s, 3H), 2.02-2.18 (m, 4H), 1.87-1.98 (m, 2H), 1.55-1.65 (m, 2H), 1.28 (s, 3H).

Compound **27c** (30 mg, 101.43 µmol, 1 *eq*)*,* compound **1f** (13.53 mg, 91.29 µmol, 0.9 *eq*)*,* methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (18.39 mg, 20.29 µmol, 0.2 *eq*) and cesium carbonate (49.57 mg, 152.15 µmol, 1.5 *eq*) were placed in a reaction flask, and the system was replaced with nitrogen for three times, then 2 mL of anhydrous dioxane was added to the mixture and reacted at 100 °C for 1.5 hours. After the reaction was completed, the reaction solution was concentrated under reduced pressure and purified by preparative thin layer chromatography (eluted successively by dichloromethane: methanol = 20: 1 and ethyl acetate: petroleum ether = 1: 0) to obtain compound **28,** MS: *m*/*z* 408.1 [M+H]⁺.

¹H NMR (400 MHz, CDCl₃) δ ppm 9.60 (s, 1H), 8.26 (s, 1H), 7.94 (s, 1H), 7.59 (s, 1H), 6.76 (s, 1H), 3.25 (s, 6H), 2.51 (s, 3H), 2.19-2.31 (m, 2H), 2.03-2.15 (m, 2H), 1.86-1.96 (m, 2H), 1.39-1.51 (m, 2H), 1.28 (s, 3H).

### Biological test data:

### Experimental embodiment 1: DNA-dependent protein kinase (DNA-PK) inhibitory activity screening experiment

The present experiment was tested in the Eurofins

### Experimental materials and methods:

Human-derived DNA-PK; Mg/ATP; GST-cMyc-p53; ethylenediaminetetraacetic acid (EDTA); Ser15 antibody; ATP: 10 µM.

### Experimental method (Eurofins Pharma Discovery Service):

DNA-PK (h) was incubated in assay buffer containing 50 nM GST-cMyc-p53 and Mg/ATP (according to the required concentration). The reaction was initiated by adding Mg/ATP mixture. After 30 minutes of incubation at room temperature, the reaction was stopped by the addition of stop solution containing EDTA. Finally, detection buffer (containing labeled anti-GST monoclonal antibody and europium-labeled anti-Ser15 antibody against phosphorylated p53) was added. The plate was then read in time-resolved fluorescence mode, and the homogeneous time-resolved fluorescence (HTRF) signal was determined according to the formula HTRF = 10000 × (Em665 nm/Em620 nm).

### Test results:

The experimental results are shown in Table 1:

**Table 1 DNA-PK kinase activity test results**

| **Testing sample** | **DNA-PK kinase inhibitory activity IC₅₀ (nM)** |
|---|---|
| Compound 1 | 39 |
| Compound 2 | 6 |
| Compound 3 | 10 |
| Compound 4 | 4 |
| Compound 5 | 16 |
| Compound 6 | 6 |
| Compound 7 | 25 |
| Compound 8 | 4 |
| Compound 9 | 3 |
| Compound 10 | 7 |
| Compound 11 | 1 |
| Compound 12 | 1 |
| Compound 13 | 6 |
| Compound 14 | 11 |
| Compound 15 | 4 |
| Compound 16 | 39 |
| Compound 17 | 7 |
| Compound 18 | 4 |
| Compound 19 | 7 |
| Compound 20 | 15 |
| Compound 21 | 11 |
| Compound 22 | 11 |
| Compound 23 | 8 |
| Compound 24 | 84 |
| Compound 25 | 6 |
| Compound 26 | 3 |
| Compound 27 | 10 |
| Compound 28 | 3 |

Conclusion: The compound of the present disclosure has significant DNA-PK kinase inhibitory activity.

### Experimental embodiment 2: Pharmacokinetic evaluation

### 1. Experimental method

The test compound was mixed with 10 % dimethyl sulfoxide/50 % polyethylene glycol 200/40 % water, vortexed and sonicated to prepare a nearly clear solution of 0.08 mg/mL, which was filtered with a microporous membrane for use. Balb/c male mice of 18 to 20 grams were selected, and the candidate compound solution was administered intravenously at a dose of 0.4 mg/kg. The test compound was mixed with 10 % dimethyl sulfoxide/50 % polyethylene glycol 200/40 % water, vortexed and sonicated to prepare a nearly clear solution of 0.2 mg/mL, which was filtered with a microporous membrane for use. Balb/c male mice of 18 to 20 grams were selected, and the candidate compound solution was orally administered at a dose of 2 mg/kg. Whole blood was collected for a certain period of time, and plasma was prepared, then drug concentration was analyzed by LC-MS/MS method, and pharmacokinetic parameters were calculated by Phoenix WinNonlin software (Pharsight, USA).

### Definition of each parameter:

IV: intravenous administration; PO: oral administration; Co: instantaneous required concentration after intravenous injection; Cₘₐₓ: maximum blood drug concentration after administration; Tₘₐₓ: time required to reach the peak drug concentration after administration; T_{1/2}: time required for the blood drug concentration to decrease by half; V_{dss}: apparent volume of distribution, refers to the proportional constant of the drug dose *in vivo* and the blood drug concentration when the drug reaches a dynamic equilibrium *in vivo.* Cl: clearance rate, refers to the apparent volume of distribution of the drug cleared *in vivo* per unit time; Tₗₐₛₜ: time at the last detection point; AUC₀₋ₗₐₛₜ: area under the drug-time curve, refers to the area surrounded by the blood drug concentration curve to the time axis; F: a measure of the speed and degree of drug absorption into the blood circulation, which is an important index for evaluating the degree of drug absorption.

The experimental results are shown in Table 2.

**Table 2 PK test results in the plasma of the embodiment compounds**

| parameter | | C₀ (nM) | Cmax (nM) | Tmax (h) | T_{1/2} (h) | V_{dss} (L/kg) | Cl (mL/min /kg) | Tₗₐₛₜ (h) | AUC₀₋ₗₐₛₜ (nM.h) | F (%) |
|---|---|---|---|---|---|---|---|---|---|---|
| Compound 9 | IV (0.4 mg/kg) | 871 | - | - | 0.241 | 1.07 | 53.4 | 2 | 311 | - |
| | PO (2 mg/kg) | - | 1077 | 0.5 | 0.479 | - | - | 4 | 1158 | 74.5 |
| Compound 11 | IV (0.4 mg/kg) | 1253 | - | - | 0.242 | 0.855 | 47.2 | 2 | 349 | - |
| | PO (2 mg/kg) | - | 1215 | 0.5 | 0.493 | - | - | 4 | 1286 | 73.7 |
| Compound 12 | IV (0.4 mg/kg) | 1276 | - | - | 0.203 | 0.794 | 45.8 | 1 | 358 | - |
| | PO (2 mg/kg) | - | 1855 | 0.5 | 0.492 | - | - | 4 | 2036 | 114 |
| Compound 15 | IV (0.4 mg/kg) | 1233 | - | - | 0.253 | 0.853 | 37.6 | 2 | 454 | - |
| | PO (2 mg/kg) | - | 1640 | 0.25 | 0.997 | - | - | 8 | 1882 | 83 |

| | | | | | | | | | | |
|---|---|---|---|---|---|---|---|---|---|---|
| "--"refers to untested or unobtained data. | | | | | | | | | | |

Conclusion: The compound of the present disclosure shows lower clearance rate and higher drug exposure, and has better pharmacokinetic properties *in vivo.*

## Claims

1. A compound represented by formula (III) or a pharmaceutically acceptable salt thereof, 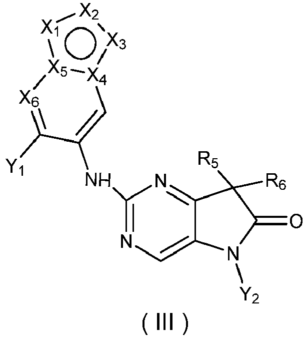 wherein,
R₅ and R₆ combining with the carbon atoms to which they are attached form 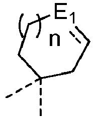 and 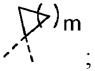 when 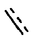 is a single bond, E₁ is selected from -O-, -S-, -C(=O)-, -S(=O)₂-, -C(R₁)(R₂)-, - N(R₃)- and 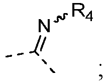 when 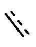 is a double bond, E₁ is selected from -C(R₁)-;
R₁ and R₂ are each independently selected from H, OH, F, Cl, Br, I, C₁₋₃ alkoxy and C₁₋₃ alkyl, and the C₁₋₃ alkoxy and C₁₋₃ alkyl are optionally substituted by 1, 2 or 3 Ra;
or, R₁ and R₂ combining with the carbon atoms to which they are attached form a cyclopropyl, cyclobutyl and oxetanyl;
R₃ is selected from C₁₋₃ alkyl-C(=O)- and C₁₋₃ alkyl, and the C₁₋₃ alkyl-C(=O)- and C₁₋₃ alkyl are optionally substituted by 1, 2 or 3 Rb;
R₄ is selected from C₁₋₃ alkoxy;
n is selected from 0, 1 and 2, provided that when E₁ is selected from -C(R₁)(R₂)-, and both R₁ and R₂ are selected from H, n is not 0;
m is selected from 1, 2 and 3;
X₁, X₂, X₃, X₄ and X₅ are each independently selected from N, C and CH, provided that at most three of X₁, X₂, X₃, X₄ and X₅ are N, and the ring formed with X₁, X₂, X₃, X₄ and X₅ is an aromatic ring;
X₆ is selected from CH and N;
Yi is selected from F, Cl, Br, I, cyclopropyl and C₁₋₃ alkyl, and the C₁₋₃ alkyl is optionally substituted by OH or 1, 2 or 3 Ra;
Y₂ is selected from cyclopropyl and C₁₋₃ alkyl, and the C₁₋₃ alkyl is optionally substituted by 1, 2, 3, 4 or 5 F;
Ra and R_{b} are each independently selected from H, F, Cl, Br, I.

2. The compound or the pharmaceutically acceptable salt thereof as claimed in claim 1, wherein, the compound represented by formula (III) or the pharmaceutically acceptable salt thereof is selected from a compound represented by formula (III-1) or a pharmaceutically acceptable salt thereof, 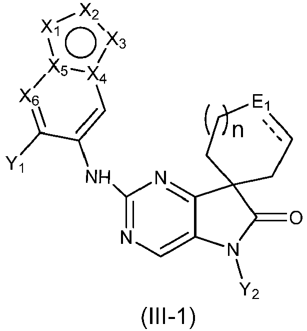 wherein, X₁, X₂, X₃, X₄, X₅, X₆, Y₁, Y₂, E₁ and n are as defined in claim 1.

3. The compound or the pharmaceutically acceptable salt thereof as claimed in claim 1, wherein, the compound represented by formula (III) or the pharmaceutically acceptable salt thereof is selected from a compound represented by formula (III-2) or a pharmaceutically acceptable salt thereof, 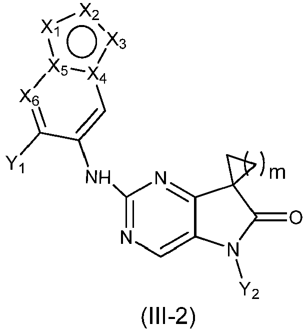 wherein, X₁, X₂, X₃, X₄, X₅, X₆, Y₁, Y₂ and m are as defined in claim 1.

4. The compound or the pharmaceutically acceptable salt thereof as claimed in any one of claims 1 to 3, wherein, X₁, X₃ and X₄ are selected from N, X₂ is selected from CH, X₅ is selected from C, and X₆ is selected from CH and N; or, X₁, X₂ and X₄ are selected from N, X₃ is selected from CH, X₅ is selected from C, X₆ is selected from CH; or, X₁, X₃ and X₅ are selected from N, X₂ is selected from CH, X₄ is selected from C, and X₆ is selected from CH; or, X₁ and X₄ are selected from N, X₂ and X₃ are selected from CH, X₅ is selected from C, X₆ is selected from CH and N.

5. The compound or the pharmaceutically acceptable salt thereof as claimed in any one of claims 1 to 4, wherein, Y₁ is selected from F, Cl, cyclopropyl, CH₃, CH₂OH, CFH₂, CF₂H and CF₃; Y₂ is selected from cyclopropyl, CH₃, CFH₂, CF₂H and CF₃.

6. The compound or the pharmaceutically acceptable salt thereof as claimed in claim 1, wherein, the compound represented by formula (III) or the pharmaceutically acceptable salt thereof is selected from a compound represented by formula (I) or a pharmaceutically acceptable salt thereof, 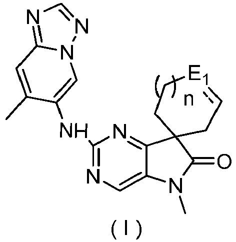 wherein, E₁ and n are as defined in claim 1.

7. The compound or the pharmaceutically acceptable salt thereof as claimed in claim 1, selecting from a compound represented by formula (II) or a pharmaceutically acceptable salt thereof, 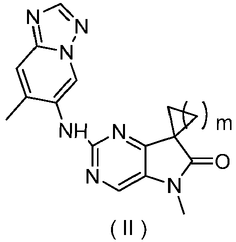 wherein, m is as defined in claim 1.

8. The compound or the pharmaceutically acceptable salt thereof as claimed in any one of claim 1, 2 or 4 to 6, wherein, 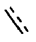 is a single bond, E₁ is selected from -O-, -S-, -C(=O)-, - S(O)₂-, -C(R₁)(R₂)-, -N(R₃)- and 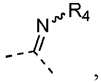 and R₁, R₂, R₃ and R₄ are as defined in claim 1.

9. The compound or the pharmaceutically acceptable salt thereof as claimed in claim 8, wherein, 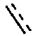 is a single bond, E₁ is selected from -O-, -C(R₁)(R₂)-, -N(R₃)- and 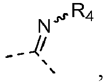 and R₁, R₂, R₃ and R₄ are as defined in claim 1.

10. The compound or the pharmaceutically acceptable salt thereof as claimed in claim 8 or 9, wherein, R₁ and R₂ are each independently selected from H, OH, F, Cl, C₁₋₃ alkoxy and C₁₋₃ alkyl, and the C₁₋₃ alkoxy and C₁₋₃ alkyl are optionally substituted by 1, 2 or 3 H or F; R₃ is selected from C₁₋₃ alkyl-C(=O)- and C₁₋₃ alkyl, and the C₁₋₃ alkyl-C(=O)- and C₁₋₃ alkyl are optionally substituted by 1, 2 or 3 H or F; R₄ is selected from C₁₋₃ alkoxy.

11. The compound or the pharmaceutically acceptable salt thereof as claimed in any one of claim 1, 2 or 4 to 6, wherein, 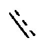 is a double bond, E₁ is selected from -C(Ri)-, R₁ is selected from H, F, Cl, Br, I, C₁₋₃ alkoxy and C₁₋₃ alkyl, and the C₁₋₃ alkoxy and C₁₋₃ alkyl are optionally substituted by 1, 2 or 3 Ra, and Ra is as defined in claim 1.

12. The compound or the pharmaceutically acceptable salt thereof as claimed in claim 11, wherein, 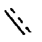 is a double bond, E₁ is selected from -C(Ri)-, R₁ is selected from H, F, C₁₋₃ alkyl, and the C₁₋₃ alkyl is optionally substituted by 1, 2 or 3 H or F.

13. The compound or the pharmaceutically acceptable salt thereof as claimed in any one of claims 8 to 10, wherein, R₁ and R₂ are each independently selected from H, OH, F, CH₃, CF₃ and CH₃O-.

14. The compound or the pharmaceutically acceptable salt thereof as claimed in claim 8 or 9, wherein, R₁ and R₂ combining with the carbon atoms to which they are attached form

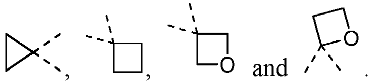

15. The compound or the pharmaceutically acceptable salt thereof as claimed in claim 8 or 9, wherein, R₃ is selected from CH₃, CH₃CH₂ and CH₃C(=O)-, and the CH₃, CH₃CH₂ and CH₃C(=O)- are optionally substituted by 1, 2 or 3 R_{b}, and R_{b} is as defined in claim 1.

16. The compound or the pharmaceutically acceptable salt thereof as claimed in claim 15, wherein, R₃ is selected from CH₃, CF₃CH₂ and CH₃C(=O)-.

17. The compound or the pharmaceutically acceptable salt thereof as claimed in any one of claims 8 to 10, wherein, R₄ is selected from CH₃O-.

18. The compound or the pharmaceutically acceptable salt thereof as claimed in any one of claim 1, 2 or 4 to 6, wherein, the structural moiety 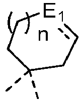 is selected from 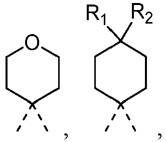 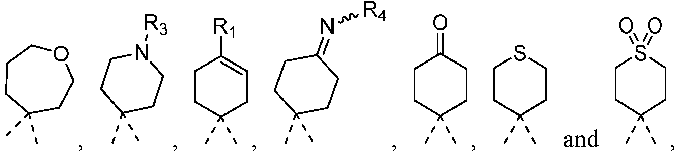 and R₁, R₂, R₃ and R₄ are as defined in any one of claim 1 or 10 to 17.

19. The compound or the pharmaceutically acceptable salt thereof as claimed in claim 18, wherein, the structural moiety 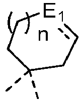 is selected from

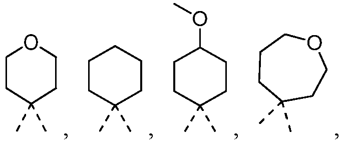

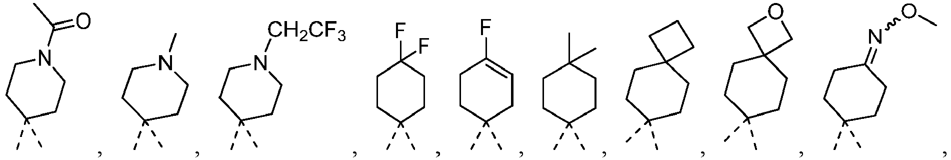

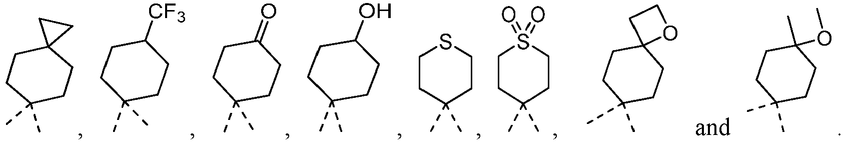

20. The compound or the pharmaceutically acceptable salt thereof as claimed in any one of claim 1 or 2, and the compound is selected from 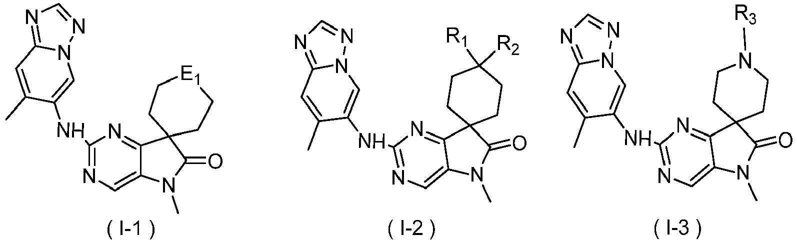 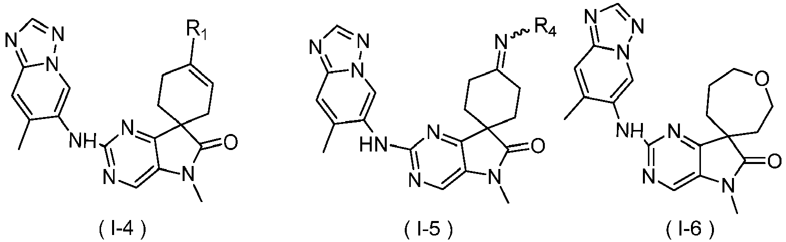 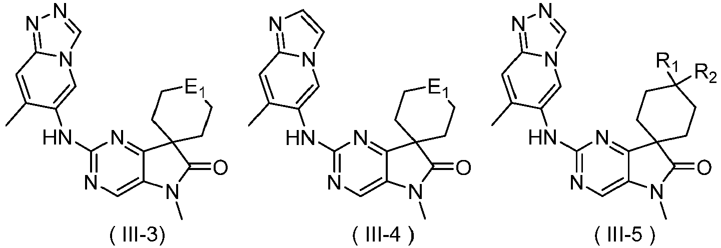 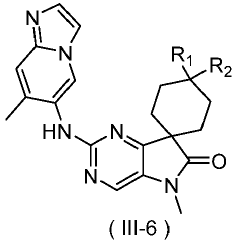 wherein, E₁, R₁, R₂, R₃ and R₄ are as defined in any one of claim 1 or 10 to 17.

21. A compound represented by the following formula or a pharmaceutically acceptable salt thereof

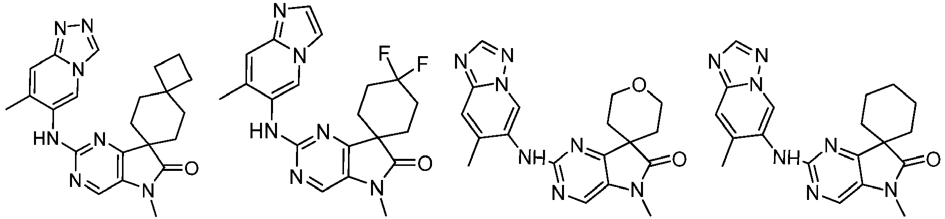

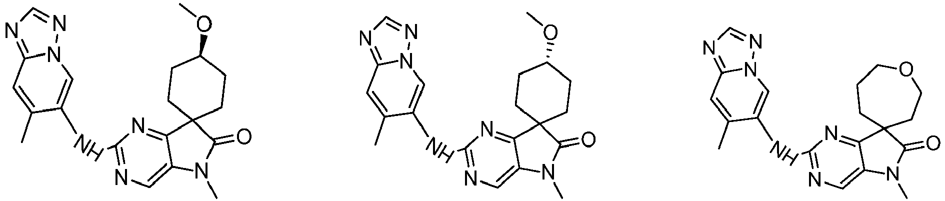

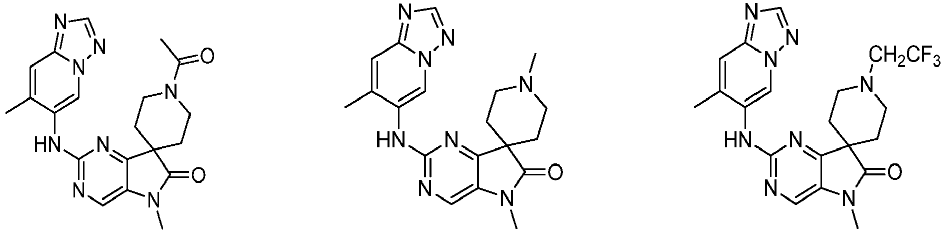

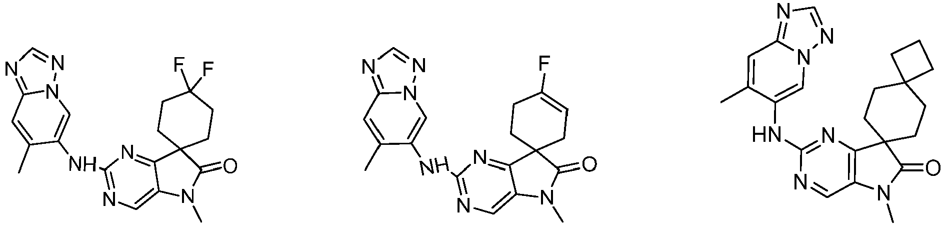

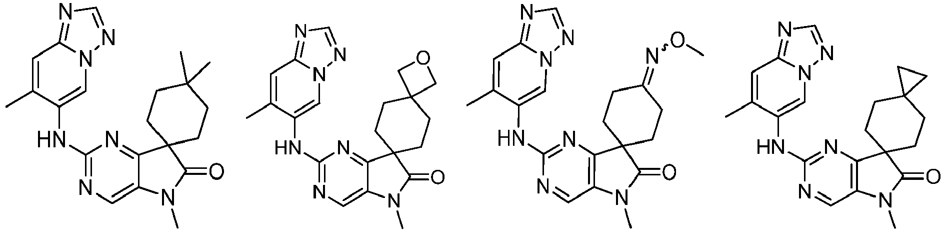

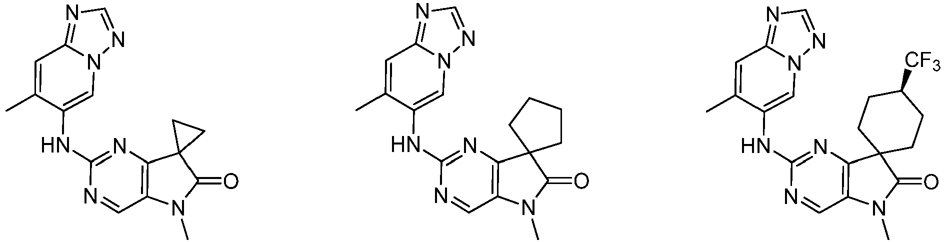

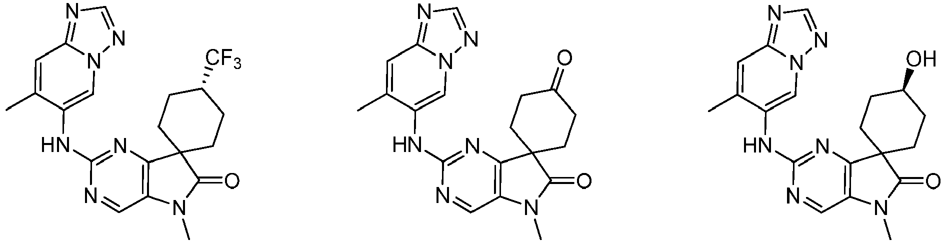

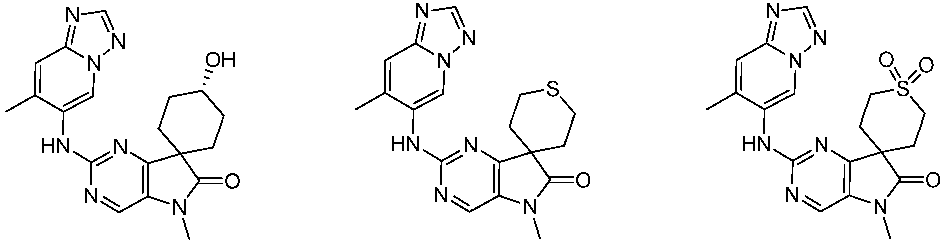

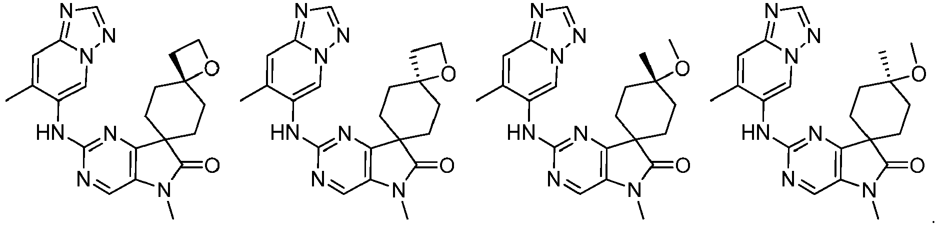

22. A use of the compound or the pharmaceutically acceptable salt thereof as claimed in any one of claims 1 to 21 in the manufacture of a medicament related to a DNA-PK inhibitor.

23. The use as claimed in claim 22, wherein, the medicament related to the DNA-PK inhibitor plays a therapeutic effect as a single medicament in tumors with defects in other DNA repair pathways.

24. The use as claimed in claim 22, wherein, the medicament related to the DNA-PK inhibitor is used in combination with chemoradiotherapy medicaments to enhance the inhibitory effect on solid tumors and hematological tumors.
